# EUROPÄISCHE PATENTSCHRIFT

(11) **EP 0 778 819 B1**
(45) Veröffentlichungstag und Bekanntmachung des Hinweises auf die Patenterteilung: **13.01.1999**
(21) Anmeldenummer: 95930520.2
(22) Anmeldetag: 24.08.1995
(51) Int. Cl.: C07C 45/51, C07C 45/56, C07C 29/00, C07C 47/02, C07C 31/12

(54) **VERFAHREN ZUR HERSTELLUNG VON N-BUTYRALDEHYD UND/ODER N-BUTANOL**
PROCESS FOR PRODUCING N-BUTYRALDEHYDE AND/OR N-BUTANOL
PROCEDE DE FABRICATION D'ALDEHYDE N-BUTYRIQUE ET/OU DE N-BUTANOL

(30) Priorität: 03.09.1994 DE 4431528
(43) Veröffentlichungstag der Anmeldung: 18.06.1997
(73) Patentinhaber: BASF AKTIENGESELLSCHAFT, 67056 Ludwigshafen (DE)
(72) Erfinder: KANAND, Jürgen, D-67098 Bad Dürkheim (DE); RÖPER, Michael, D-67157 Wachenheim (DE); PACIELLO, Rocco, D-67098 Bad Dürkheim (DE); THOME, Alfred, D-67346 Speyer (DE)
(86) Internationale Anmeldenummer: EP9503358
(87) Internationale Veröffentlichungsnummer: WO9607630

(56) Entgegenhaltungen:
- EP-A- 0 257 411
- DE-A- 4 400 837
- US-A- 3 391 192
- US-A- 3 714 270
- CHEMISTRY LETTERS, Bd. 12, 1982 TOKYO JP, Seiten 1987-1988, K. TAKABE ET AL. 'A Simple Conversion of N,N-Dimethyl-2-Alkenylamine to 2-Alkenal'
- Advanced Org. Chem., J. March, 3rd ed., 1985, John Wiley & Sons

## Beschreibung

Die vorliegende Erfindung betrifft ein neues Verfahren zur Herstellung von n-Butyraldehyd und/oder n-Butanol sowie die Verwendung des nach diesem Verfahren hergestellten n-Butyraldehyds zur Herstellung von 2-Ethylhexanol. Weiterhin betrifft die Erfindung ein Verfahren zur Herstellung von 2-Ethylhexanol aus n-Butyraldehyd.

n-Butyraldehyd und n-Butanol sind Großprodukte der chemischen Industrie und finden vielseitige Verwendung. n-Butyraldehyd wird weltweit in Mengen von über 4 Millionen t/Jahr produziert und dient u.a. als Ausgangsmaterial zur Herstellung von Weichmacheralkoholen. n-Butanol wird in großem Umfang als Lösungsmittel, beispielsweise für Lacke, eingesetzt.

n-Butyraldehyd wird heute großtechnisch praktisch ausschließlich durch die Hydroformylierung von Propen hergestellt, wozu verschiedene Verfahren, die im wesentlichen Kobalt- oder Rhodium-Hydroformylierungskatalysatoren verwenden, benutzt werden (Kirk-Othmer: Encyclopedia of Chemical Technology, 4. Aufl., Band 4, S. 741 - 746, John Wiley & Sons, New York 1992).

n-Butanol ist eines der mengenmäßig wichtigsten Folgeprodukte des n-Butyraldehyds und wird aus diesem durch Hydrierung gewonnen. Andere Verfahren zur Herstellung von n-Butanol, wie die Hydrierung von Crotonaldehyd, der wiederum durch Aldolkondensation von Acetaldehyd erzeugt wird, sind heute nur noch von historischem Interesse oder haben, wie die mikrobiologische Erzeugung von n-Butanol durch die Vergärung von Melasse, nur regionale Bedeutung (Kirk-Othmer: Encyclopedia of Chemical Technology, 4. Aufl., Band 4, S. 694 - 696, John Wiley & Sons, New York 1992). Diese Verfahren, insbesondere die Hydroformylierung von Propen, erfordern hohe Investitionen, beispielsweise für die Errichtung von Hochdruckanlagen für die Kobalt-katalysierte Hydroformylierung oder für den Kauf des teuren Rhodium-Katalysators, die Anlagen für dessen Handhabung bei der Hydroformylierung und zur Aufarbeitung verbrauchter, Rhodium-haltiger Katalysatorlösungen. Des weiteren müssen zur Herstellung von n-Butyraldehyd nach dem Hydroformylierungsverfahren Synthesegasanlagen zur Verfügung stehen, die das zur Hydroformylierung benötigte Synthesegas liefern. Ein weiterer Nachteil des Hydroformylierungsverfahrens ist der hohe Zwangsanfall des Nebenprodukts Isobutyraldehyd, der wirtschaftlich, aufgrund seiner mengenmäßig beschränkten Weiterverwendungsmöglichkeiten, niedrig bewertet wird.

1,3-Butadien ist eine Grundchemikalie, die in großen Mengen in Steamcrackern erzeugt und aus dem C₄-Schnitt von Steamcrackern extraktiv, beispielsweise mittels N-Methylpyrrolidon, isoliert wird. Obwohl 1,3-Butadien in großen Mengen zur Verfügung steht und ein sehr preiswerter Rohstoff ist, wurde bislang noch kein großtechnisch anwendbares Verfahren zur Herstellung von n-Butyraldehyd oder n-Butanol auf der Basis von 1,3-Butadien entwickelt. Ein Grund hierfür ist sowohl in der Neigung des 1,3-Butadiens zu Dimerisierungs- und Polymerisationsreaktionen als auch die Bildung von Gemischen aus 1,2- und 1,4-Addukten bei Additionsreaktionen zu sehen. Ursächlich für dieses chemische Verhalten ist das Vorliegen zweier konjugierter Doppelbindungen im 1,3-Butadienmolekül (Kirk-Othmer: Encyclopedia of Chemical Technology, 4. Aufl., Band 4, S. 676 - 683, John Wiley & Sons, New York 1992).

Es ist aus US-A 33 91 192 bekannt, daß Amine mit 1,3-Butadien in Gegenwart von Alkalimetallamiden zu den entsprechenden Allylaminen reagieren. Falk et al. (J. Org. Chem. 37, 4243 (1972)) untersuchten die Lithiumamid-katalysierte Addition von Aminen an Butadien in Abhängigkeit vom Lösungsmittel und Amin. Kakuno und Hattori (J. Catal. 85 (1984) 509) beschrieben die katalysierte Umsetzung von Butadien und Aminen mit Hilfe fester Basenkatalysatoren wie MgO oder CaO. Die Reaktionsmischung wird dabei in einem Kreisgasreaktor über ein Festbett bei einem Gesamtdruck von 100 Torr geleitet. Gemäß US-A 46 75 307 sind stark basische Hydrotalcite als Katalysatoren für die Hydroaminierung geeignet.

Auch Übergangsmetallkomplexe wurden als Katalysatoren für die Addition von Aminen an 1,3-Butadien genutzt. Watanabe et al. (Kenkyu Hokoku - Asahi Garasu Kogyo Gijiutsu Shoreikai 38, 111 (1981)) beschreiben die Reaktion von verschiedenen primären und sekundären Aminen mit Butadien in Gegenwart von Palladium- und Platin-Komplexen. Während sekundäre Amine je nach Art des Katalysators vorwiegend Butenylamine liefern, erhält man mit primären Aminen Gemische aus 1 : 1-, 1 : 2- und 1 : 4-Addukten. Die JP-A 71/19 925, JP-A 71/19 926 und JP-A 72/25 321 betreffen die katalytische Herstellung von Alkenylaminen mit Hilfe von Palladiumverbindungen und chelatisierenden Phosphinliganden. Von Takahashi et al. (Bull. Chem. Soc. Jap. 45, 1183 (1972)) wird diese Reaktion zusätzlich in der Anwesenheit von Natriumphenolat/Phenol durchgeführt. Ein anderer Cokatalysator bei der palladiumkatalysierten Hydroaminierung ist Triethylammoniumiodid (Armbruster et. al., Organomet. 5, 234 (1986)), das zur Erhöhung der Selektivität der Reaktion zum Monoaddukt eingesetzt wird. Die Addition von Ammoniak zur Herstellung von 1-Aminobut-2-en gelingt gemäß US-A 41 20 901 mit Palladiumkomplexen in primären oder sekundären Alkoholen als Lösungsmittel. Eine Aminierung von 1,3-Dienen in Gegenwart optisch aktiver Phosphorverbindungen beschreibt die US-A 42 04 997.

Ligandeneffekte einer Rhodium-katalysierten Reaktion von Butadien mit Aminen wurden von Baker und Halliday (Tetrahedron Lett. 2773 (1972)) untersucht. Aus der EP-A 176 398 ist bekannt, daß die Umsetzung von sekundären Aminen mit 1,3-Butadien in Gegenwart von wasserlöslichen Rhodiumkomplexen gelingt. Als Cokatalysatoren finden hier trisulfonierte Phosphine Anwendung. Herrmann et al. (Angew. Chem. 102, 408 (1990)) setzen für die Reaktion von Isopren mit Dimethylamin entsprechende wasserlösliche Platinkomplexe ein.

Neben den genannten Übergangsmetallen sind auch Hydroaminierungen in Gegenwart von Nickel-, Kobalt- und Iridiumverbindungen bekannt. So wurde von Baker et. al. (J. Chem. Soc. Perkin II, 1511 (1974)) die Reaktion von cyclischen und aliphatischen sekundären Aminen mit Butadien in Gegenwart katalytischer Mengen Nickelacetylacetonat und Phosphitliganden untersucht. Die gleiche Reaktion mit Hilfe von Kobalt- oder Iridiumkatalysatoren führt zu Gemischen aus 1 : 2- und 1 : 1-Addukten.

Zur Isomerisierung von Allylaminen zu Enaminen wurden bereits eine Reihe von Reagenzien untersucht.

Die Isomerisierung von 1-N-Pyrrolidino-2-propen mit basischen heterogenen Oxidkatalysatoren wie MgO, CaO oder BaO ist von Hattori et al. (J. Catal. 65, 245 (1980)) beschrieben. Hubert (J. Chem. Soc. (C), 2048 (1968)) führt die Umlagerung von Allylaminen in Gegenwart von geträgertem Kaliumamid auf Aluminiumoxid durch.

Außer den genannten Heterogenkatalysatoren wurden auch homogene Katalysatoren zur Isomerisierung von Allylaminen in flüssiger Phase eingesetzt.

Im organischen Medium lösliche starke Basen wie beispielsweise Kalium-tert.-butanolat wurden von Price et al. (Tetrahedron Lett. 69 (1962)), Sauer (Tetrahedron Lett. 2863 (1966)) und Martinez (Tetrahedron 34, 3027 (1978)) zur Umwandlung von Allylaminen zu Enaminen untersucht.

Neben diesen basenkatalysierten Reaktionen sind auch Umsetzungen mit Übergangsmetallverbindungen beschrieben.

Isomerisierungen von sekundären und tertiären Allylaminen mit Rhodium-Diphosphin-Komplexen zu Enaminen bzw. Iminen sind von Otsuka et al. (J. Am. Chem. Soc. 106, 5208 (1984)) und Schmid et al. (Helv. Chim. Acta 73, 1258 (1990); Helv. Chim. Acta 74, 370 (1991)) beschrieben. Aus der US-A 48 61 890, EP-A 006 8506 und EP-A 25 7411 und von Otsuka (Org. Synth. 67, 33 (1989)) ist bekannt, daß β,γ-ungesättigte Amine mit Rhodium-Diphosphin-Komplexen zu Enaminen isomerisieren, die nach Hydrolyse in ihre entsprechenden Aldehyde überführt werden.

Eine Cobalt-Phosphinkomplex-katalysierte Umlagerung von Allylaminen wird in JP-A 79/59 07 und von Otsuka (J. Am. Chem. Soc. 100, 3949 (1978)) gelehrt.

Neben den genannten können auch Molybdän- (Tatsumi et al., J Organomet. Chem. 252, 105 (1983)) oder Rutheniumverbindungen (EP-A 398 132; Doering et al., J. Am. Chem. Soc. 107, 428 (1985)) eingesetzt werden.

Die direkte einstufige Umwandlung von Allylaminen zu den entsprechenden Aldehyden oder Alkoholen ist nicht bekannt.

Der vorliegenden Erfindung lag somit die Aufgabe zugrunde, ein großtechnisch anwendbares, wirtschaftliches Verfahren zur Herstellung von n-Butyraldehyd und/oder n-Butanol zu finden, das es ermöglicht, diese Produkte mit hoher Ausbeute und Selektivität herzustellen, insbesondere sollte die Menge der im Verfahren gebildeten Nebenprodukte gering sein oder diese Nebenprodukte selbst gesuchte Handelsprodukte sein. Weiterhin sollte das Verfahren flexibel sein, um zu ermöglichen, daß wahlweise n-Butyraldehyd und/oder n-Butanol, entsprechend dem Bedarf für diese Verbindungen, hergestellt werden können. Die Ausübung des Verfahrens sollte nicht auf das Vorhandensein seiner Synthesegasanlage angewiesen sein und ohne Hochdruckanlagen auskommen.

2-Ethylhexanol wird großtechnisch über eine Aldolreaktion von n-Butyraldehyd mit anschließender Hydrierung des Aldolproduktes hergestellt (Kirk-Othmer: Encyclopedia of Chemical Technology, 4th Edition, 1991, Vol. 1, S. 893; Ullmanns Encyklopädie der techn. Chemie, 4. Auflage, 1974, Band 4, S. 214). Der Alkohol dient zur Herstellung des Weichmachers Bis-(2-ethylhexyl)-phthalat. Eine weitere Aufgabe bestand somit darin, ein Verfahren bereitzustellen, das es erlaubt, n-Butyraldehyd ohne aufwendige Reinigung in 2-Ethylhexanol zu überführen.

Dementsprechend wurde ein Verfahren zur Herstellung von n-Butyraldehyd und/oder n-Butanol gefunden, das dadurch gekennzeichnet ist, daß man
a) 1,3-Butadien mit einem Amin der Formel I

   R¹R²NH I,

   in der die Reste R¹ und R² unabhängig voneinander für Wasserstoff, gegebenenfalls substituierte aliphatische oder cycloaliphatische Reste, Aryl- oder Aralkylreste stehen oder zu einem Brückenglied, das Heteroatome enthalten kann, verbunden sind, bei erhöhter Temperatur und bei erhöhtem Druck in Gegenwart einer Verbindung eines Elementes aus der Gruppe VIIIb des Periodensystems der Elemente, in Gegenwart eines Alkalimetallamids oder eines basischen Metalloxids, zu einem Gemisch der Addukte der Formeln II 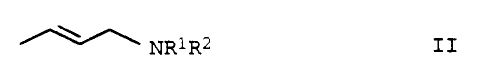 und III 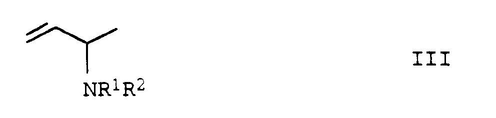 umsetzt,
b) das Addukt III zum Addukt II isomerisiert,
c) das Addukt II in Gegenwart eines homogenen oder heterogenen Übergangsmetallelement-Katalysators in flüssiger Phase oder in Gegenwart eines heterogenen, Übergangsmetallelement-haltigen Katalysators in der Gasphase zum Enamin der Formel IV 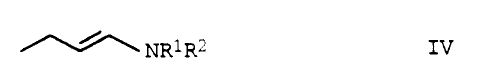 isomerisiert, und
d) aus diesem Enamin IV durch dessen Umsetzung mit Wasserstoff und Wasser oder Wasser in Gegenwart eines homogenen oder heterogenen Übergangsmetallelement-Katalysators in flüssiger Phase, in Gegenwart eines heterogenen Übergangselementhaltigen Katalysators in der Gasphase, in Gegenwart einer Säure oder in Gegenwart eines der genannten Katalysatoren und einer Säure n-Butyraldehyd und/oder n-Butanol erzeugt, das Amin I freisetzt und das freigesetzte Amin I wieder in die Umsetzung gemäß Teilreaktion a) zurückführt.

Weiterhin wurde ein Verfahren zur Herstellung von 2-Ethylhexanol gefunden.

Das erfindungsgemäße Verfahren zur Herstellung von n-Butyraldehyd und/oder n-Butanol setzt sich somit aus 4 Teilreaktionen a) bis d) zusammen. Die Teilreaktionen c) und d) können wahlweise einzeln, nacheinander, in mindestens 2 Verfahrensstufen oder praktisch simultan in einer einzigen Verfahrensstufe durchgeführt werden. Ähnliches gilt für die Teilreaktionen a) und b), wobei die Isomerisierung des Addukts III zum Addukt II gemäß Teilreaktion b) nach Rückführung des Addukts III in die Verfahrensstufe der Addition des Amins R¹R²NH an 1,3-Butadien simultan mit der Addition gemäß Teilreaktion a) verläuft. Dadurch wird es auf einfache Weise möglich, die Verfahrensbedingungen für das erfindungsgemäße Verfahren an die örtlichen Gegebenheiten des Standortes, an dem eine Anlage zur Ausübung des Verfahrens installiert wird, anzupassen, beispielsweise indem man vor Ort bereits bestehende Anlagenteile in die Anlage für das erfindungsgemäße Verfahren integriert.

Der Ausdruck "Verfahrensstufe" wird in dieser Anmeldung für eine Anlageneinheit gebraucht, in der jeweils eine einzelne der Teilreaktionen a) bis d) an dem oder den in dieser Anlageneinheit eingesetzten Katalysator oder Katalysatoren abläuft oder in der mehrere, insbesondere 2 dieser Teilreaktionen, nebeneinander an dem oder den in dieser Anlageneinneit verwendeten Katalysator oder Katalysatoren ablaufen. Die Hydrolyse bzw. die kombinierte Hydrolyse/Hydrierung des Enamins IV gemäß Teilreaktion d) wird dabei, sofern in dieser Anmeldung nichts anderes erwähnt wird, als eine einzelne Teilreaktion betrachtet.

Ist der in einer Anlageneinheit verwendete Katalysator oder jeder der in einer Anlageneinheit verwendeten Katalysatoren in der Lage, unter den dort angewandten Reaktionsbedingungen beispielsweise die Isomerisierung des Addukts II zum Enamin IV gemäß Teilreaktion c) und die Hydrolyse oder Hydrierung des Enamins IV zu n-Butyraldehyd und/oder n-Butanol gemäß Teilreaktion d) zu katalysieren, so daß keine strenge räumliche Trennung des Ablaufs dieser Teilreaktionen in der Anlageneinheit festgestellt werden kann, so wird in dieser Anmeldung von der Durchführung der Teilreaktionen c) und d) in einer einzigen Verfahrensstufe gesprochen. Eine Anlageneinheit kann dabei sowohl einen einzigen Reaktor als auch mehrere, in Reihe geschaltete Reaktoren umfassen, die mit dem gleichen oder gegebenenfalls mit verschiedenen Katalysatoren befüllt sind und in der gleichen Betriebsweise und bei gleichen oder verschiedenen Temperatur- und Druckbedingungen betrieben werden. Als Betriebsweise wird dabei jeweils das Arbeiten in flüssiger Phase unter Anwendung eines Homogenkatalysators oder das Arbeiten in flüssiger Phase unter Anwendung eines Heterogenkatalysators oder das Arbeiten in der Gasphase verstanden. Daraus folgt, daß in dieser Anmeldung beispielsweise dann nicht von einer "Umsetzung in einer einzigen Verfahrensstufe" gesprochen wird, wenn in den einzelnen, aufeinanderfolgenden Reaktoren Katalysatoren angewandt werden, die allein zur Katalyse einer bestimmten Teilreaktion befähigt sind oder wenn in diesen Reaktoren mit unterschiedlichen Betriebsweisen gearbeitet wird.

Im folgenden wird das erfindungsgemäße Verfahren näher erläutert:

In Stufe a) wird 1,3-Butadien in Gegenwart eines Katalysators mit dem Amin R¹R²NH I gemäß Gleichung (1) 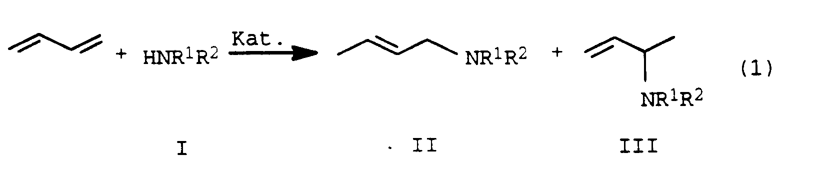 zu den Mono-Addukten der Formel II und III sowie bei Verwendung eines primären Amins oder Ammoniaks weiterhin zu Gemischen von Di- und Triaddukten sowie zu Telomeren umgesetzt. Im entstandenen Mono-Addukt II kann die Doppelbindung sowohl in der cis- als auch trans-Form vorliegen, dies ist für den weiteren Ablauf des Verfahrens aber nicht erheblich. Die Addukte II und III entstehen dabei je nach Reaktionsbedingungen und angewandtem Katalysator im allgemeinen in einem Molverhältnis II : III von 1 : 1 bis 20 : 1.

Die Art des in der Umsetzung eingesetzten Amins R¹R²NH I ist in der Regel für das Verfahren nicht kritisch. Es kommen sowohl Ammoniak wie auch primäre und sekundäre Amine in Betracht. Die Amine können eine Vielzahl verschiedener Reste R¹ bzw. R² tragen. So kommen aliphatische Reste wie Alkylreste, insbesondere C₁- bis C₂₀-Alkylreste, sowie C₂-C₂₀-Alkenylreste ebenso in Betracht wie Cycloalkylreste, insbesondere C₄-C₁₀-Cycloalkyl, und C₄-C₁₀-Cycloalkenylreste. Die nicht-cyclischen Reste können geradkettig oder verzweigt sein. Die aliphatischen Reste können unter den Reaktionsbedingungen inerte Substituenten tragen, vorzugsweise 1 bis 2 Substituenten, von denen C₁-C₁₀-Alkoxygruppen, Aminogruppen sowie Hydroxygruppen zu nennen sind. Weiterhin können die Reste R¹ bzw. R² für Arylgruppen, vorzugsweise C₆-C₁₀-Aryl wie Phenyl stehen, wobei die Arylreste durch inerte Reste wie C₁-C₄-Alkyl substituiert sein können. Auch Aralkylreste, vorzugsweise C₇-C₁₁-Aralkyl wie Benzyl, kommen als Reste am Amin I in Betracht.

Die Reste R¹ und R² können auch zu einer Brücke unter Ausbildung eines stickstoffhaltigen Cyclus verknüpft sein. Die Zahl der Brückenatome beträgt bevorzugt 3 bis 6. Die Brücke kann Heteroatome wie Sauerstoff oder Stickstoff enthalten. Sie kann gesättigt oder ungesättigt oder Bestandteil eines aromatischen Rings sein. Weiterhin kann sie inerte Substituenten wie C₁-C₄-Alkylgruppen tragen.

Besonders bevorzugt sind Ammoniak, C₁-C₆-N-Alkylanilin, sowie Amine, in denen die Reste R¹ und R² unabhängig voneinander für verzweigte oder geradkettige C₁-C₆-Alkylreste, C₂-C₆-Alkenylreste, C₄-C₇-Cycloalkylreste stehen.

Im folgenden werden beispielhaft einige erfindungsgemäß zu verwendende Amine aufgeführt:

Methylamin, Ethylamin, n-Propylamin, iso-Propylamin, n-Butylamin, iso-Butylamin, sek.-Butylamin, tert.-Butylamin, iso-Pentylamin, 3-Methyl-2-butylamin, n-Hexylamin, Octylamin, 2-Ethylhexylamin, Decylamin, tert.-Hexylamin, 1,1,3,3-Tetramethylbutylamin, Allylamin, 2-Butenylamin, 3-Pentenylamin, Hexylamin, n-Heptylamin, Cyclopentylamin, Cyclohexylamin, Methylcyclohexylamin, Cyclooctylamin, Cyclodecylamin, Benzylamin, 2-Phenylethylamin, 4-Methoxyphenylethylamin, Anilin, Toluidin, 2-Diethylaminoethylamin, Dimethylaminopropylamin, 2-Aminoethanol, 1-Amino-2-propanol, 3-Aminopropanol-1, 2-Aminobutanol-1, Dimethylamin, Diethylamin, Di-n-propylamin, Di-iso-propylamin, Di-n-butylamin, Di-iso-butylamin, Di-sek.-butylamin, Di-n-pentylamin, Di-isopentylamin, Di-n-hexylamin, Di-2-ethylhexylamin, N-Methylbutylamin, N-Ethylbutylamin, Di-2-methoxyethylamin, N-Methylcyclohexylamin, N-Ethylcyclohexylamin, N-Methylethylamin, Dicyclohexylamin, N-Ethylanilin, Diamylamin, Di-n-octylamin, Allylmethylamin, 2-Butenylethylamin, Dialkylamin, N-Methylbenzylamin, Allylmethallylamin, Pyrrolidin, Piperidin, 4-Methylpiperidin, Morpholin, 2,6-Dimethylmorpholin, Imidazol, 2-Methylimidazol, 4-Methylimidazol, Piperazin, 1-Ethylpiperazin, Pyrazol, Ethylendiamin, 1,3-Diaminopropan, 1,2-Propyldiamin, Neopentandiamin, Hexamethylendiamin, Diethylentriamin.

Als Katalysatoren können in der Stufe a) eine Vielzahl von Übergangsmetallelement-Katalysatoren, beispielsweise Verbindungen des Palladiums, Platins, Nickels, Rhodiums, Kobalts und Iridiums, oder stark basische Verbindungen, beispielsweise Metallamide, Metallalkoholate und Hydrotalcite, eingesetzt werden.

In einer Ausgestaltung des erfindungsgemäßen Verfahrens kann die Addition des Amins I mittels eines homogen im Reaktionsmedium gelösten oder heterogenisierten Übergangsmetallelementkatalysators, der ein Element aus der Gruppe VIIIb des Periodensystems der Elemente, wie Palladium, Platin, Nickel, Rhodium, Kobalt und Iridium, vorzugsweise Palladium und Nickel, enthält, vorgenommen werden.

Zweckmäßigerweise werden diese Übergangsmetallelementkatalysatoren, insbesondere die Palladium- und Nickel-Katalysatoren, in Form ihrer homogen im Reaktionsmedium löslichen Komplexe mit z.B. Phosphin-, 2,2'-Bipyridin- oder 1,10-Phenanthrolin-Liganden eingesetzt. Im erfindungsgemäßen Verfahren können zu diesem Zweck zur Komplexierung der Gruppe der VIIIb-Metalle, insbesondere des Palladiums und Nickels, eine Vielzahl unterschiedlicher Phosphin-, 2,2'-Bipyridin- oder 1,10-Phenanthrolin-Liganden verwendet werden. Als Liganden können sowohl einzähnige oder mehrzähnige, insbesondere zweizähnige, Phosphin-Liganden verwendet werden. Geeignete Phosphin-Liganden sind z.B. Trialkylphosphine, Triarylphosphine, Alkyldiarylphosphine, Aryldialkylphosphine, Aryldiphosphine, Alkyldiphosphine und Arylalkyldiphosphine. Die Alkylgruppen-tragenden Phosphin-Liganden können gleiche oder verschiedene C₁- bis C₁₀-, vorzugsweise C₁- bis C₆-Alkyl- oder -Cycloalkylgruppen enthalten. Die Arylgruppentragenden Phosphin-Liganden können gleiche oder verschiedene C₆- bis C₁₂-Arylgruppen, insbesondere die Phenyl- oder Naphthylgruppe aber auch Diphenylgruppen enthalten. Neben den genannten Phosphinen sind auch wasserlösliche, insbesondere sulfonierte Phosphine wie Natriumtriphenylphosphintrisulfonat, verwendbar. Weiterhin können Phosphin-Liganden zur Komplexierung der Gruppe der VIIIb-Elemente eingesetzt werden, die heterocycloaliphatische Gruppe wie Pyrrolidin-, Imidazolidin-, Piperidin-, Morpholin-, Oxazolidin-, Piperazin- oder Triazolidin-Gruppe oder heteroaromatischen Gruppen, wie Pyrrol-, Imidazol-, Oxazol-, Indol-, Pyridin-, Chinolin-, Pyrimidin-, Pyrazol-, Pyrazin-, Pyridazin- oder Chinoxalin-Gruppen gemeinsam mit anderen Alkyl- oder Aryl-Gruppen tragen. Die Alkyl- oder Arylgruppen der Liganden können unsubstituiert sein oder unter den Reaktionsbedingungen inerte Substituenten, wie C₁- bis C₆-Alkyl-, Nitro-, Cyano- oder Sulfonat-Gruppen tragen.

Prinzipiell besteht keine Beschränkung für die Anwendbarkeit derartiger Liganden zur Komplexierung der Gruppe der VIIIb-Elemente, insbesondere des Palladiums und Nickels im erfindungsgemäßen Verfahren. Vorzugsweise werden aber aus Kostengründen solche Liganden eingesetzt, die auf einfache Weise hergestellt werden können. Eine lediglich beispielhafte Aufzählung derartiger Liganden wird im folgenden gegeben:
Trimethylphosphin, Triethylphosphin, Tripropylphosphin, Triisopropylphosphin, Tributylphosphin, Trioctylphosphin, Tridecylphosphin, Tricyclopentylphosphin, Tricyclohexylphosphin, Triphenylphosphin, Tritolylphosphin, Cyclohexyldiphenylphosphin, Tetraphenyldiphosphinomethan, 1,2-Bis(diphenylphosphino)ethan, Tetramethyldiphosphinomethan, Tetraethyldiphosphinomethan, 1,3-Bis(diphenylphosphino)propan, 1,4-Bis(diphenylphosphino)-butan, Bis(di-tert.-butyl-diphosphino)methan, 1,2-Bis-(dimethylphosphino)ethan, 1,2-Bis(diethylphosphino)ethan, 1,2-Bis(dipropylphosphino)ethan, 1,2-Bis(diisopropylphosphino)-ethan, 1,2-Bis(dibutylphosphino)ethan, 1,2-Bis(di-t-butyl-phosphino)ethan, 1,2-Bis(dicyclohexlphosphino)ethan, die Salze des Triphenylphosphintrisulfonats oder des Triphenylphosphinmonosulfonats, sowie die in EP-A 279 081, EP-A 311 619, WO 90/06 810 und EP-A 71 281 beschriebenen Bisphosphin-Liganden. Außer nach den in den zuvor genannten Patentanmeldungen beschriebenen Verfahren können die Alkyl- bzw. Arylphosphin-Liganden nach an sich herkömmlichen Methoden, beispielsweise gemäß den in Houben-Weyl, Methoden der Organischen Chemie, Band XII/1, 4. Auflage, S. 17 - 65 und S. 182 - 186, Thieme, Stuttgart, 1963 und Band E 1, 4. Auflage, S. 106 - 199, Thieme, Stuttgart, 1982 angegebenen Verfahren hergestellt werden.

Außer Phosphin-Liganden können im erfindungsgemäßen Verfahren vorteilhaft auch 2,2'-Bipyridin- oder 1,10-Phenanthrolin-Liganden der Alkyl- oder Aryl-substituierte oder anellierte 2,2'-Bipyridin- oder 1,10-Phenanthrolin-Derivate, die die für die Komplexbildungseigenschaft der 2,2'-Bipyridin- oder 1,10-Phenanthrolin-Liganden ursächliche (-N=C-C=N-)-Gruppierung enthalten, beispielsweise 2,2'-Bichinolin, 4,7-Diphenyl-1,10-phenanthrolin, 4,5-Diazafluoren, Dipyrido[3,2-a:2',3'-c]phenazin, 2,2',6',2''-Terpyridin u.ä., eingesetzt werden. Diese Liganden sind zum Teil im Handel erhältlich, z.B. 2,2'-Bipyridin oder 1,10-Phenanthrolin oder können nach den in Synthesis 1, (1976) oder Aust. J. Chem. 23, 1023 (1970) angegebenen Methoden hergestellt werden.

Die im erfindungsgemäßen Verfahren für die Teilreaktion a) anwendbaren Komplexe der Elemente der Gruppe VIIIb, insbesondere des Palladiums und Nickels, können sowohl in situ im Reaktionsgemisch erzeugt oder nach ihrer gesonderten Herstellung der Reaktionsmischung zugesetzt werden. Zur in-situ-Erzeugung dieser Komplexe wird im allgemeinen so vorgegangen, daß man Verbindungen der Gruppe VIIIb-Elemente, z.B. deren Halogenide, vorzugsweise deren Chloride, Bromide oder Iodide, die Nitrate, Cyanide oder Sulfate oder Komplexverbindungen dieser Metalle, wie Acetylacetonate, Carboxylate, Carbonylkomplexe oder Olefinkomplexe, wie Ethen oder Butadien-Komplexe, zusammen mit dem betreffenden Liganden der Reaktionsmischung zuführt, worauf sich die erfindungsgemäß in Teilreaktion a) anwendbaren Komplexe in der Reaktionsmischung bilden. Im allgemeinen wird hierbei der Komplexbildner bezüglich des Gruppe VIIIb-Elementes in einem Molverhältnis von 2 bis 200, vorzugsweise von 2 bis 10, insbesondere von 2 bis 4 zugegeben.

Im allgemeinen wird bei der Addition des Amins R¹R²NH an 1,3-Butadien in Stufe a) des erfindungsgemäßen Verfahrens bei Anwendung der genannten Gruppe der VIIIb-Element-Komplex-Katalysatoren, insbesondere der Palladium-Komplex-Katalysatoren ein Molverhältnis von 1,3-Butadien/Gruppe VIIIb-Element von 100 : 1 bis 100 000 : 1, vorzugsweise von 200 : 1 bis 10 000 : 1 und besonders bevorzugt von 400 : 1 bis 5 000 : 1 eingestellt, wobei dieses Molverhältnis im Falle der kontinuierlichen Durchführung des Verfahrens die stationäre 1,3-Butadien-Konzentration in der flüssigen Reaktionsmischung wiedergibt.

Das Molverhältnis Amin R¹R²NH/1,3-Butadien kann bei dieser Verfahrensausgestaltung in weiten Grenzen gewählt werden und ist in der Regel nicht kritisch. Beispielsweise kann das an 1,3-Butadien zu addierende Amin I außer als Reagenz auch als Lösungsmittel für den Komplexkatalysator fungieren. Im allgemeinen wird deshalb im erfindungsgemäßen Verfahren in der Teilreaktion a) ein Amin/1,3-Butadien-Molverhältnis von 0,5 : 1 bis 10 : 1, vorzugsweise von 1 : 1 bis 5 : 1 und besonders bevorzugt von 1 : 1 bis 2 : 1 angewandt, wobei sich diese Angaben im Falle der kontinuierlichen Ausgestaltung des Verfahrens auf die stationäre 1,3-Butadien-Konzentration in der flüssigen Reaktionsmischung beziehen.

Die Addition des Amins R¹R²NH an 1,3-Butadien gemäß Teilreaktion a) des erfindungsgemäßen Verfahrens mit Hilfe der genannten Komplexkatalysatoren wird vorzugsweise in flüssiger Phase durchgeführt. Im allgemeinen wird der Katalysator gelöst im flüssigen Reaktionsmedium vorgelegt und 1,3-Butadien in flüssiger oder gasförmiger Form zusammen mit dem Amin I in die Reaktionsmischung eingeleitet. Als Reaktionsmedium kann das an 1,3-Butadien zu addierende Amin oder ein unter den Reaktionsbedingungen inertes Lösungsmittel, vorzugsweise ein hochsiedendes Lösungsmittel, dienen. Als Lösungsmittel eignen sich beispielsweise Kondensationsprodukte, die im Zuge der Umsetzung entstehen, wie Aminooctadiene, ferner Ether, wie Dibutylether, Diethylenglykoldibutylether, niedermolekulare Polyethylenglykolether sowie Alkohole, wie Methanol oder Butanol.

Bei der diskontinuierlichen Ausgestaltung des Verfahrens wird die Umsetzung im allgemeinen in einem Rührautoklaven durchgeführt. Die dabei gebildeten Addukte der Formeln II und III werden anschließend zweckmäßigerweise aus der Reaktionsmischung destillativ abgetrennt, wobei der das VIIIb-Element, insbesondere Palladium oder Nickel, enthaltende Homogenkatalysator im Sumpf der Destillation, im hochsiedenden Lösungsmittel gelöst, zurückbleibt. Die so im Destillationssumpf verbliebene Katalysatorlösung kann für weitere Umsetzungen wiederverwendet werden.

Bei der kontinuierlichen Ausgestaltung des Verfahrens wird das 1,3-Butadien vorzugsweise in flüssiger Form unter Druck in die das Amin R¹R²NH und den homogen gelösten Übergangsmetallelementkomplex-Katalysator sowie gegebenenfalls ein hochsiedendes Lösungsmittel enthaltende Reaktionsmischung eingespeist. Die Umsetzung wird vorteilhaft in einem Rühr- oder Schlaufenreaktor oder vorzugsweise in einer Reaktorkaskade durchgeführt. Nicht umgesetztes 1,3-Butadien wird dabei vorteilhaft im Kreis geführt. Das Amin wird vorteilhaft bei der Umsetzung der Reaktionsmischung kontinuierlich zudosiert, so daß es immer in stöchiometrischem Überschuß vorhanden ist.

Nach einer weiteren kontinuierlichen Ausgestaltung des erfindungsgemäßen Verfahrens kann das 1,3-Butadien gasförmig durch das den Katalysator enthaltende flüssige Reaktionsmedium geleitet werden, wobei nicht umgesetztes 1,3-Butadien dazu verwendet wird, die bei der Umsetzung mit dem Amin gebildeten Addukte der Formeln II und III aus der Reaktionsmischung zu strippen. Das Amin I kann dabei entsprechend seinem Verbrauch bei der Umsetzung der Reaktionsmischung zudosiert werden.

Die Addition des Amins R¹R²NH an 1,3-Butadien in Gegenwart der genannten Komplexe der Gruppe der VIIIb-Elemente, insbesondere des Palladiums oder Nickels, wird im allgemeinen bei einer Temperatur von 20 bis 180°C, vorzugsweise von 50 bis 150°C und besonders bevorzugt von 80 bis 120°C und bei einem Druck von vorzugsweise 6 bis 50 bar und besonders bevorzugt unter Eigendruck durchgeführt.

Vorteilhaft können im erfindungsgemäßen Verfahren zur Addition des Amins R¹R²NH an 1,3-Butadien in Teilreaktion a) heterogenisierte Komplexkatalysatoren verwendet werden, vorzugsweise solche, in denen das VIIIb-Element, insbesondere das Palladium oder Nickel, an polymere Matrizes fixiert ist. Solche polymeren Matrizes können Harze, wie Styrol-Divinylbenzol-Harze oder Phenol-Formaldehyd-Harze sein, an die die betreffenden Chelatliganden, also Phosphine, 1,10-Phenanthroline oder 2,2'-Bipyridine, gebunden sind, welche wiederum mit den VIIIb-Elementen, insbesondere dem Palladium oder Nickel, Komplexe bilden und diese derart quasi immobilisieren. Als heterogene Matrizes zur Immobilisierung der Gruppe der VIIIb-Element-Komplexe, insbesondere der Palladium- und Nickel-Komplexe, können auch anorganische Trägermaterialien, nach vorausgegangener Hydrophobisierung und chemischer Modifizierung ihrer Oberflächen mittels organischer Reagenzien dienen. Solche heterogenisierten, polymer gebundenen VIIIb-Element-Komplexe, insbesondere Palladium- und Nickel-Komplexe, sind beispielsweise nach dem Verfahren von Zhuangyu et al. (Reactive Polymers 9, 249 (1988)) erhältlich. Immobilisierte Phosphin-Komplexe der VIIIb-Elemente sind z.B. nach den Verfahren von Hartley, Adv. Organomet. Chem. 15, 189 (1977), F.R. Harley "Supported Metal Complexes", Riedel, Dordrecht 1985, K. Smith, "Solid Supports and Catalysis in Organic Synthesis", Ellis Horwood, Prentice Hall, N.Y. 1992; C.H. Pittman "Polymer supported Reactions in Organic Synthesis, S. 249, Wiley, Chichester 1980 und C.H. Pittmann J. Am. Chem. Soc. 98, 5407 (1976) sowie Ann. N.Y. Acad. Sci. 245, 15 (1977) erhältlich. Der Vorteil der Anwendung solcher heterogenisierter Katalysatoren liegt insbesondere in der leichteren und schonenderen Abtrennbarkeit des Katalysators von den Reaktionsprodukten. Dieser kann in einem Festbett angeordnet und von der Reaktionsmischung durchströmt werden oder aber auch in der Reaktionsmischung suspendiert und nach beendeter Umsetzung, mechanisch abgetrennt werden.

In einer weiteren Ausgestaltung des Verfahrensschrittes a) kann die Reaktion zwischen 1,3-Butadien und dem Amin R¹R²NH I in Gegenwart eines Alkalimetallamids vorgenommen werden. Bevorzugt sind dabei die den Aminen R¹R²NH entsprechenden Amide. Diese können in situ oder vom Reaktionsgemisch getrennt hergestellt werden, wobei die in situ-Herstellung aus praktischen Gründen bevorzugt wird. Die Herstellung erfolgt aus dem Amin R¹R²NH und einer starken Base, welche in der Regel einen pkₐ-Wert von über 20 aufweist. Als starke Basen kommen Organoalkalimetallverbindungen wie Phenylnatrium, n-Butyllithium, sek.-Butyllithium, Methyllithium, Naphthalide des Lithiums, Natriums und Kaliums, Graphitverbindungen wie C₈K und C₂₄K ebenso in Betracht wie die Hydride von Lithium, Natrium und Kalium. Auch die Alkalimetalle selbst können mit den Aminen zu den entsprechenden Amiden umgesetzt werden. Die Alkalimetallamide können in katalytischen und stöchiometrischen Mengen eingesetzt werden, wobei katalytische Mengen von 0,001 bis 0,1 mol pro Mol Amin bevorzugt werden. Es hat sich als vorteilhaft erwiesen, Butadien in eine vorgelegte Mischung aus Amin R¹R²NH und dem entsprechenden Amid einzuleiten und die gebildeten Addukte II und III abzudestillieren. Hierzu können an sich übliche Reaktoren wie Blasensäulen und Schlaufenreaktoren verwendet werden, es können aber auch Rührkesselkaskaden verwendet werden. Die Reaktion kann kontinuierlich oder diskontinuierlich erfolgen. Auch für diese Verfahrensvariante gelten die Druck- und Temperaturbereiche, wie sie für die Umsetzung von Butadien und Amin R¹R²NH in Gegenwart einer Verbindung eines Elementes der Gruppe VIIIb des Periodensystems angegeben wurden.

Weiterhin kann die Herstellung der Addukte II und III unter den oben genannten Bedingungen in Gegenwart von basischen Metalloxiden wie MgO, CaO, SrO, La₂O₃ und Hydrotalcit erfolgen. Die Metalloxide können in Substanz oder auf inerten Trägermaterialien verwendet werden.

Anstelle von reinem 1,3-Butadien können im erfindungsgemäßen Verfahren auch 1,3-Butadien-haltige Kohlenwasserstoffströme als Rohstoff eingesetzt werden. Derartige Kohlenwasserstoffströme fallen beispielsweise als sogenannter C₄-Schnitt in Steamcrackern an. Zweckmäßigerweise werden diese Kohlenwasserstoffströme vor ihrem Einsatz im erfindungsgemäßen Verfahren von gegebenenfalls darin enthaltenen acetylenischen oder allenischen Kohlenwasserstoffen durch partielle Hydrierung (Weissermel, Arpe: Industrielle Organische Chemie; 3. Auflage, VCH Verlagsgesellschaft, Weinheim 1988) befreit. Die 1,3-Butadien-haltigen Kohlenwasserstoffströme können sodann in analoger Weise wie reines 1,3-Butadien in Teilreaktion a) des erfindungsgemäßen Verfahrens eingetragen werden. Zweckmäßigerweise werden die in diesen Kohlenwasserstoffströmen enthaltenen gesättigten oder monoolefinischen Kohlenwasserstoffe, die bei der Umsetzung in Teilreaktion a) nicht reagiert haben, aus dem Reaktionsaustrag von Teilreaktion a) entfernt, beispielsweise mittels eines Gas-Flüssigkeit-Abscheiders. Die bei der Umsetzung dieser Kohlenwasserstoffströme in Teilreaktion a) des erfindungsgemäßen Verfahrens erhaltenen Addukte der Formeln II und III können, wie im folgenden beschrieben, auf die gleiche Weise zu n-Butyraldehyd und/oder n-Butanol weiterverarbeitet werden, wie die mit reinem 1,3-Butadien in Teilreaktion a) erzeugten Addukte II und III.

Der Reaktionsaustrag aus Teilreaktion a) des erfindungsgemäßen Verfahrens enthält im allgemeinen neben nicht umgesetzten 1,3-Butadien die Addukte II und III sowie gegebenenfalls, insbesondere bei Anwendung von Übergangsmetallkatalysatoren in Teilreaktion a), Isomere des betreffenden Aminooctadiens, von denen im folgenden unter der Sammelbezeichnung Aminooctadien gesprochen wird. Das Aminooctadien entsteht durch Telomerisation in einer Nebenreaktion. Neben diesen Bestandteilen kann der Reaktionsaustrag aus Teilreaktion a) noch geringe Mengen an anderen Nebenprodukten enthalten, beispielsweise Octatrien und Vinylcyclohexen. Die Bildung dieser Nebenprodukte kann durch die Art und Weise der Reaktionsführung in Teilreaktion a), beispielsweise durch die Wahl des 1,3-Butadien/Amin R¹R²NH-Verhältnisses in der Reaktionsmischung, die Wahl der Reaktionstemperatur und des Drucks beeinflußt und gewünschtenfalls minimiert werden.

Das zur Herstellung von n-Butyraldehyd und/oder n-Butanol im erfindungsgemäßen Verfahren benötigte Addukt ist das 1-Aminobut-2-en der Formel II, das für die Herstellung der Zielverbindungen im erfindungsgemäßen Verfahren von seinem im Reaktionsaustrag mitenthaltenen Isomeren 3-Aminobut-1-en der Formel III abgetrennt werden kann. Da je nach den gewählten Reaktionsbedingungen verschiedene Molverhältnisse von 1-Aminobut-2-en II/3-Aminobut-1-en III gebildet werden, wäre das erfindungsgemäße Verfahren großtechnisch nicht wirtschaftlich, wenn es nicht gelänge, das 3-Aminobut-1-en in wirtschaftlicher Weise in das gewünschte 1-Aminobut-2-en umzuwandeln.

Zu diesem Zweck wird das Addukt III zunächst aus dem im Reaktionsaustrag von Teilreaktion a) enthaltenen, isomeren Addukt II abgetrennt. Dies kann vorteilhaft so geschehen, daß der Reaktionsaustrag aus Teilreaktion a), nach vorheriger Abtrennung nicht umgesetzten 1,3-Butadiens, z.B. in einem Gas-Flüsssigkeit-Abscheider oder in einer Druckkolonne in eine Destillationsapparatur geleitet und darin durch fraktionierte Destillation abgetrennt wird.

Bei dieser fraktionierten Destillation können auch die im Reaktionsaustrag von Teilreaktion a) enthaltenen Nebenprodukte, 1,3-Butadien-Dimere und -Trimere, vom Addukt II abgetrennt werden. Da diese Nebenprodukte sich im weiteren Gang des erfindungsgemäßen Verfahrens im allgemeinen nicht störend auswirken, kann deren Abtrennung auch unterbleiben. Es kann bei der Destillation auch so verfahren werden, daß man außer dem Addukt III nur einen Teil der Nebenprodukte, insbesondere die olefinischen 1,3-Butadien-Dimere abtrennt, hingegen andere Nebenprodukte, insbesondere das Aminooctadien gemeinsam mit dem Addukt II in den nachfolgenden Teilreaktionen weiterverarbeitet, wobei aus diesen Nebenprodukten der Teilreaktion a) als Endprodukte Octanole entstehen, die als Weichmacheralkohole begehrt sind.

Die destillative Abtrennung des leichter flüchtigen Addukts III vom Addukt II gelingt auf einfache Weise z.B. in herkömmlichen Destillationskolonnen. Das vom Addukt II abgetrennte Addukt III kann dann, ebenso wie das nicht umgesetzte 1,3-Butadien in die Verfahrensstufe der Teilreaktion a) des erfindungsgemäßen Verfahrens zurückgeführt werden. Die Rückführung des Addukts III in die Verfahrensstufe der Teilreaktion a) des erfindungsgemäßen Verfahrens bewirkt die Isomerisierung des Addukts III zum Addukt II in dieser Verfahrensstufe und führt letztendlich zur Unterdrückung der Neubildung des unerwunschten Addukts III, so daß bei Anwendung dieser Kreislauffahrweise in der Gesamtbilanz dieses Kreisprozesses praktisch nur das erwünschte Addukt II, nicht jedoch dessen unerwünschtes Isomer III gebildet wird.

Die Isomerisierung des Addukts III kann statt durch dessen Rückführung in die Verfahrensstufe der Teilreaktion a) des erfindungsgemäßen Verfahrens auch in einer separaten Isomerisierungsstufe bewerkstelligt werden, indem man das vom Addukt II abgetrennte Addukt III z.B. durch einen mit einem der in Teilreaktion a) anwendbaren Katalysatoren beschickten Reaktor leitet, den Austrag dieses Reaktors, der aus dem darin gebildeten Isomerisierungsgemisch aus Addukt III und Addukt II besteht, beispielsweise destillativ in Addukt II und Addukt III auftrennt, das neu gebildete Addukt II im weiteren Gang des erfindungsgemäßen Verfahrens zu n-Butyraldehyd und/oder n-Butanol und/oder 2-Ethylhexanol weiterverarbeitet und das Addukt III wieder in den Isomerisierungsreaktor zurückführt.

Die Isomerisierung des Addukts III zu Addukt II im Isomerisierungsreaktor kann in An- oder Abwesenheit eines Lösungsmittels erfolgen. Vorzugsweise wird bei dieser Umsetzung ohne Lösungsmittel gearbeitet. Wird die Isomerisierung in Gegenwart eines Lösungsmittels durchgeführt, werden im allgemeinen hochsiedende Lösungsmittel wie Ether, beispielsweise Di- oder Triethylenglykoldimethylether, Di- oder Triethylenglykoldibutylether, hochsiedende aromatische oder aliphatische Kohlenwasserstoffe oder halogenierte aliphatische oder aromatische Lösungsmittel, z.B. Dichlorbenzol, eingesetzt. Die Verwendung niedrig siedender Lösungsmittel ist ebenfalls möglich, erfordert in der Regel aber einen erhöhten Aufwand bei der destillativen Auftrennung des Auftrags aus dem Isomerisierungsreaktor in die Addukte II und III.

Im weiteren Verlauf des erfindungsgemäßen Verfahrens zur Herstellung von n-Butyraldehyd und/oder n-Butanol wird das Addukt II in der Teilreaktion c) katalytisch zum Enamin der Formel IV isomerisiert, das anschließend in der Teilreaktion d) in Gegenwart von Wasser zu n-Butyraldehyd hydrolysiert und/oder in Gegenwart von Wasser und Wasserstoff in n-Butanol umgewandelt wird und/oder in Gegenwart von Wasser zu n-Butyraldehyd hydrolysiert. Die Teilreaktionen c) und d) können im erfindungsgemäßen Verfahren wahlweise sukzessive in zwei Verfahrensstufen oder sukzessive in einem einzelnen Reaktor oder besonders vorteilhaft einstufig, in einer einzigen Verfahrensstufe durchgeführt werden. Beide Teilreaktionen c) und d) können sowohl in der Gasphase als auch in flüssiger Phase stattfinden.

Wie erwähnt werden die Teilreaktion c), die Isomerisierung des Addukts II zum Enamin IV und d), dessen Umsetzung mit Wasser oder Wasserstoff und Wasser zu n-Butyraldehyd und/oder n-Butanol sowohl in einer einzigen Verfahrensstufe oder in mehreren Verfahrensstufen durchgeführt. Diese Verfahrensstufen umfassen folglich die folgenden chemischen Umsetzungen gemäß Reaktionsgleichung (2)

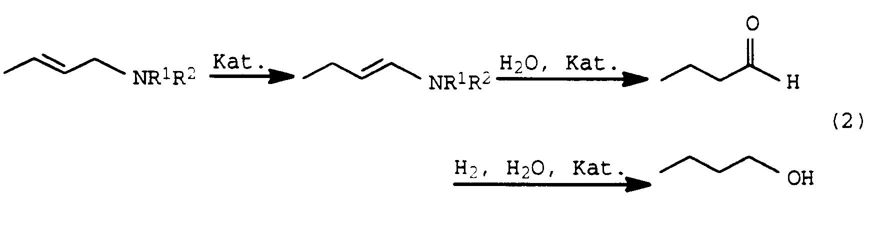

Der jeweils letzte Reaktionsschritt, d.h. die Hydrolyse des Enamins IV zu n-Butyraldehyd einerseits oder die kombinierte Hydrolyse/Hydrierung des Enamins IV zu n-Butanol, kann durch die Wahl der Reaktionsbedingungen, insbesondere durch die Wahl des Katalysators oder der Säure und die Wahl der bei der Umsetzung zur Verfügung gestellten Menge der Reaktanten Wasser und Wasserstoff, so gesteuert werden, daß wahlweise das Endprodukt n-Butyraldehyd oder n-Butanol selektiv gebildet wird oder daß Gemische dieser beiden Wertprodukte als Endprodukt des erfindungsgemäßen Verfahrens entstehen.

Es wurde überraschenderweise gefunden, daß die Katalysatoren, welche die Isomerisierung des Addukts II zu Enamin IV katalysieren, im allgemeinen auch gut als Katalysatoren für die Hydrolyse des Enamins IV zu n-Butyraldehyd oder für die kombinierte Hydrolyse/Hydrierung des Enamins IV zu n-Butanol geeignet sind. Dementsprechend können bei der besonders bevorzugten Ausführungsform des erfindungsgemäßen Verfahrens, also der Durchführung der Teilreaktionen c) und d) in einer einzigen Verfahrensstufe, sowohl für die Herstellung des Endproduktes n-Butyraldehyd als auch für die Herstellung des Endproduktes n-Butanol die gleichen Katalysatoren Anwendung finden.

Sowohl die Isomerisierung des Addukts II zu Enamin IV als auch die Hydrolyse des Enamins IV zu n-Butyraldehyd können in der Gasphase oder in flüssiger Phase ausgeführt werden. Bei der Durchführung dieser Reaktionsschritte in einer einzigen Verfahrensstufe in flüssiger Phase können sowohl homogene als auch heterogene Katalysatoren angewandt werden. Wird in der Gasphase gearbeitet, werden im allgemeinen heterogene Katalysatoren bevorzugt.

Als Homogenkatalysatoren zur Isomerisierung des Addukts II zum Enamin IV und dessen Hydrolyse oder kombinierte Hydrolyse/Hydrierung zu n-Butyraldehyd und/oder n-Butanol in einer einzigen Verfahrensstufe können eine Vielzahl von Übergangsmetallelementverbindungen eingesetzt werden, insbesondere solche, die Elemente aus der VI, VII. und VIII. Nebengruppe des Periodensystems der Elemente, vorzugsweise Chrom, Molybdän, Wolfram, Rhenium, Kobalt, Nickel, Ruthenium, Rhodium, Palladium, Platin, Osmium und/oder Iridium enthalten.

Als Katalysatoren eignen sich beispielsweise die Salze dieser Übergangsmetalle, insbesondere deren im Reaktionsmedium löslichen Halogenide, Nitrate, Sulfate, Phosphate, Carboxylate, beispielsweise deren C₁- bis C₂₀-Carboxylate, wie Formiate, Acetate, Propionate, 2-Ethylhexanoate, ferner die Citrate, Tartrate, Malate, Malonate, Maleinate oder Fumarate, Sulfonate, beispielsweise Methansulfonate, Benzolsulfonate, Naphthalinsulfonate, Toluolsulfonate oder Trifluormethansulfonate, Cyanide, Tetrafluoroborate, Perchlorate oder Hexafluorophosphate, weiterhin lösliche Salze der Oxosäuren dieser Metalle, insbesondere die Alkalimetall-, Erdalkalimetall- oder Oniumsalze, wie Ammonium-, Phosphonium-, Arsonium- oder Stiboniumsalze, der Vanadiumsauerstoffsäuren, Rheniumsauerstoffsäuren oder der Perrheniumsäure oder die Anhydride dieser Säuren, insbesondere das Dirheniumheptoxid, lösliche anorganische Komplexverbindungen dieser Elemente, insbesondere deren Aquo-, Ammin-, Halogeno-, Phosphin-, Phosphit-, Cyano- oder Amino-Komplexe sowie die Komplexe dieser Übergangsmetalle mit Chelatbildnern, wie Acetylaceton, Dioximen, beispielsweise Diacetyldioxim, Furildioxim oder Benzildioxim, Ethylendiamintetraessigsäure, Nitrilotriessigsäure, Nitrilotriethanol, Harnstoffen oder Thioharnstoffen, Bisphosphinen, Bisphosphiten, Bipyridinen, Terpyridinen, Phenanthrolinen, 8-Hydroxychinolin, Kronenethern oder Polyalkylenglykolen, sowie Organometallverbindungen dieser Übergangsmetallelemente, beispielsweise Carbonylkomplexe wie RuCl₂(CO)₂(PPh₃)₂, HRuCl(CO)(PPh₃)₃, HRuCl(CO)(Hexyldiphenylphosphin)₃, RuH₂(CO)(PPh₃)₃, HRh(CO)(PPh₃)₃ oder IrCl(CO)(PPh₃)₃ - die Abkürzung PPh₃ bedeutet Triphenylphosphin -, weiterhin RuH₂(PPh)₃, HRhCl(PPh₃)₃, Fe₂(CO)₉ oder Fe₃(CO)₁₂, Organotrioxorhenium(VII)-Verbindungen wie C₁- bis C₄-Alkyltrioxorhenium(VII), insbesondere Methyltrioxorhenium(VII), Cyclopentadienyltrioxorhenium(VII) oder Phenyltrioxorhenium(VII).

Bevorzugte salzartige Homogenkatalysatoren sind die Halogenide, insbesondere die Chloride, Nitrate, Sulfate, Carboxylate und Cyanide des Rhodiums, Rutheniums, Palladiums, Platins, Iridiums, Rheniums und Vanadiums sowie die Alkalimetall-, Erdalkalimetall-, Ammonium-, Alkylammonium-, Arylammonium-, Arylphosphonium- und Alkylphosphoniumsalze der Vanadiumsäuren, insbesondere deren Monovanadate sowie die entsprechenden Salze der Rheniumsäuren, insbesondere deren Rhenate (IV), Rhenate (VI) und Perrhenate.

Ein geeigneter Homogenkatalysator ist weiterhin das Dirheniumheptoxid (Re₂O₇).

Anorganische Komplexverbindungen, die bevorzugt im erfindungsgemäßen Verfahren zur Durchführung der Teilreaktionen c) und d) eingesetzt werden, sind z.B. Rutheniumtrichlorid, Rhodiumtrichlorid oder Iridiumhexaquoditosylat.

Übergangsorganometallelement-Verbindungen, die erfindungsgemäß bevorzugt als Homogenkatalysatoren zur Durchführung der Teilreaktionen c) und d) verwendet werden, sind z.B. Carbonylkomplexe, wie HRh(PPh₃)₃(CO), HRuCl(CO)(PPh₃)₃ oder H₂Ru(CO)₂(PPh₃), besonders bevorzugt RuCl₂(CO)₂(PPh₃)₃, sowie Organotrioxorhenium-Verbindungen der Formel V 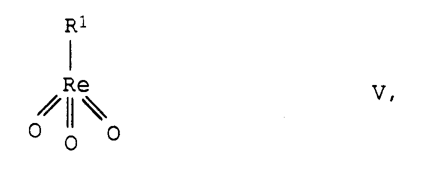 in der R¹ eine C₁- bis C₁₀-Alkylgruppe, eine unsubstituierte oder eine mit 1 bis 5 C₁- bis C₄-Alkylgruppen substituierte Cyclopentadienylgruppe, eine C₆- bis C₁₀-Aryl- oder eine C₇- bis C₁₁-Aralkylgruppe ist. Zur Herstellung dieser Organotrioxorhenium-Verbindungen wird auf die in Angew. Chem. 100, 420 (1988), Angew. Chem. 103, 183 (1991), J. Organomet. Chem. 297, C 5 (1985), Angew. Chem. 100, 1269 (1988) und J. Organomet. Chem. 382, 1 (1990) angegebenen Verfahren verwiesen.

Bevorzugte Homogenkatalysatoren für die Durchführung der Teilreaktionen c) und d) in einer einzigen Verfahrensstufe sind Komplexe der genannten Übergangsmetallelemente, insbesondere des Kobalts, Nickels, Rhodiums, Rutheniums, Palladiums, Platins und Iridiums mit einzähnigen oder mehrzähnigen, insbesondere zweizähnigen, Phosphin- oder Phosphit-Liganden und/oder mit stickstoffhaltigen Liganden, für deren Eigenschaft als Komplexbildner die (-N=C-C=N-)-Struktureinheit ursächlich ist, beispielsweise 2,2'-Bipyridin oder 1,10-Phenanthrolin, sowie die durch Substitution oder Anellierung von den genannten Heterocyclen abgeleiteten Liganden.

Geeignete Phosphin-Liganden sind beispielsweise die zur Durchführung der Teilreaktion a) des erfindungsgemäßen Verfahrens geeigneten und bei der Beschreibung dieser Teilreaktion in dieser Anmeldung genannten Phosphin-Liganden, auf die hiermit verwiesen wird. Geeignete 2,2'-Bipyridin- oder 1,10-Phenanthrolin-Liganden sind beispielsweise die zur Durchführung der Teilreaktion a) des erfindungsgemäßen Verfahrens geeigneten und bei der Beschreibung dieser Teilreaktion genannten 2,2'-Bipyridin- oder 1,10-Phenanthrolin-Liganden sowie deren am angegebenen Ort genannten Derivate und Strukturanaloge, auf die hiermit verwiesen wird.

Geeignete Phosphit-Liganden sind z.B. Trialkylphosphite, Alkyldiarylphosphite, Triarylphosphite, Alkyl-bis-phosphite, Aryl-bis-phosphite, Alkyl-aryl-bis-phosphite. Die Alkylgruppen-tragenden Phosphit-Liganden können gleiche oder verschiedene C₁- bis C₁₀-, vorzugsweise C₁- bis C₆-Alkyl- oder Cycloalkylgruppen enthalten. Die Arylgruppen-tragenden Phosphit-Liganden können gleiche oder verschiedene C₆- bis C₁₂-Arylgruppen, insbesondere die Phenyl- oder Naphthylgruppe, aber auch die Diphenylgruppe enthalten. Weiterhin können Phosphit-Liganden zur Komplexierung der Übergangsmetalle eingesetzt werden, die heterocycloaliphatische Gruppen, wie Pyrrolidin-, Imidazolidin-, Piperidin-, morpholin-, Oxazolidin-, Piperazin- oder Triazolidin-Gruppen oder heteroaromatische Gruppen, wie Pyrrol-, Imidazol-, Oxazol-, Indol-, Pyridin-, Chinolin-, Pyrimidin-, Pyrazol-, Pyrazin-, Pyridazin- oder Chinoxazolin-Gruppen neben Alkyl- oder Arylgruppen tragen. Die Alkyl- oder Arylgruppen der Phosphit-Liganden können unsubstituiert sein oder unter den Reaktionsbedingungen inerte Substituenten, wie C₁- bis C₄-Alkoxy-, Di-C₁- bis C₄-alkylamino-, C₁- bis C₆-Alkyl-, Hydroxy-, Nitro-, Cyano- oder Sulfonat-Gruppen tragen. Die Sulfonat-substituierten Phosphit-Liganden und deren Komplexe sind im allgemeinen wasserlöslich. Geeignete Phosphit-Liganden sind z.B. Trimethylphosphit, Triethylphosphit, Tripropylphosphit, Triisopropylphosphit, Tributylphosphit, Tricyclopentylphosphit, Tricyclohexylphosphit, Triphenylphosphit sowie die in EP-A 472 071, EP-A 213 639, EP-A 214 622, DE-A 2 733 796, EP-A 2261, EP-A 2821, EP-A 9115, EP-A 155 508, EP-A 353 770, US-A 4 318 845, US-A 4 204 997 und US-A 4 362 830 beschriebenen Mono- und Bisphosphit-Liganden.

Bei der Durchführung der Teilreaktionen c) und d) mit homogen im Reaktionsmedium löslichen Phosphin- oder Phosphit-Komplexen als Katalysatoren kann es sich als vorteilhaft erweisen, zusätzlich ein Phosphin oder Phosphit, vorzugsweise das im eingesetzten Homogenkatalysator als Ligand dienende Phosphin oder Phosphit, zur Reaktionsmischung zu geben. Ein solcher Zusatz kann eine Verlängerung der Standzeit des Homogenkatalysators bewirken und darüber hinaus die Selektivität der Isomerisierung des Addukts II zum Enamin IV als auch die Selektivität bei der kombinierten Hydrolyse/Hydrierung des Enamins IV zu n-Butanol und somit letztendlich die Selektivität des Gesamtverfahrens verbessern. Einen ähnlichen vorteilhaften Effekt kann der Zusatz von Kohlenmonoxid zum Reaktionsgemisch auslösen, insbesondere bei Verwendung carbonylgruppenhaltiger Übergangsmetallelementkomplexe als Homogenkatalysatoren.

Obgleich zur Herstellung des Endproduktes n-Butyraldehyd ein Zusatz von Wasserstoff zur Reaktionsmischung nicht erforderlich ist, kann die Zuführung geringer Mengen Wasserstoff, gegebenenfalls zusammen mit der Zufuhr geringer Mengen von Kohlenmonoxid bei Verwendung carbonylgruppenhaltiger Homogenkatalysatoren, zu einer Verlängerung der Standzeit dieser Homogenkatalysatoren führen. Praktischerweise kann zu diesem Zweck Synthesegas eingesetzt werden.

Zur Erzielung der obengenannten Wirkungen wird im allgemeinen das Phosphin oder Phosphit bezüglich des Phosphin- oder Phosphit-Komplexes des Übergangsmetallelementes in einer 2 bis 100 molaren, vorzugsweise in einer 2 bis 20 molaren und besonders bevorzugt in einer 2 bis 10 molaren Menge zugesetzt. Wird der als Homogenkatalysator dienende Übergangsmetallelementkomplex in situ im Reaktionsgemisch erzeugt, so setzt man zweckmäßigerweise einen entsprechend hohen Überschuß an Phosphin- oder Phosphit-Ligand bezüglich des betreffenden Übergangsmetallelementes ein.

Die homogen im Reaktionsmedium löslichen Übergangsmetallkatalysatoren werden im allgemeinen bezüglich des dem Reaktor zugeführten Addukts II in Mengen von 0,0001 bis 0,5 mol-%, vorzugsweise 0,0002 bis 0,2 mol-%, eingesetzt. Es versteht sich für den Fachmann von selbst, daß die zuzugebende Menge an Homogenkatalysator von der katalytischen Aktivität des jeweils verwendeten Homogenkatalysators abhängig ist. Je nach Art des eingesetzten Homogenkatalysators kann somit vorteilhaft auch eine höhere oder geringere Katalysatormenge der Reaktionsmischung zugesetzt werden. Zweckmäßigerweise wird die optimale Menge für den jeweils verwendeten Homogenkatalysator in einem Vorversuch ermittelt.

Die Durchführung der Teilreaktionen c) und d) in einer einzigen Verfahrensstufe mit Hilfe der genannten Homogenkatalysatoren kann diskontinuierlich, z.B. in Rührkesseln oder kontinuierlich, z.B. in Rohrreaktoren oder Schlaufenreaktoren, bei Temperaturen von im allgemeinen 80 bis 150°C und bei einem Druck von im allgemeinen 5 bis 100 bar, vorzugsweise von 10 bis 60 bar vorgenommen werden. Die Isomerisierung des Addukts II zum Enamin IV und dessen Umsetzung zu n-Butyraldehyd und/oder n-Butanol in einer einzigen Verfahrensstufe kann in An- oder Abwesenheit von zugesetzten Lösungsmitteln, wie aliphatischen oder aromatischen Kohlenwasserstoffen, z.B. Toluol, Benzol oder Cyclohexan, Alkoholen, z.B. Butanole, insbesondere n-Butanol, höhere Fettalkohole oder Glykole, Ethern, z.B. Dibutylether, Tetrahydrofuran, Dioxan oder niedermolekulare Polyalkylenglykole, halogenierte aliphatischen oder aromatischen Kohlenwasserstoffen, z.B. Chloroform, Dichlormethan, Chlorbenzol, Dichlorbenzol, Sulfoxiden oder Sulfonen, z.B. Dimethylsulfoxid oder Sulfolan erfolgen.

Werden bei der einstufigen Umsetzung des Addukts II zu den Endprodukten n-Butyraldehyd und/oder n-Butanol keine weiteren Lösungsmittel zugesetzt, so bewirken die Reaktanten selbst, also das Addukt II das Enamin IV und das erfindungsgemäß zur Hydrolyse des Enamins IV eingesetzte Wasser, als auch die Endprodukte der Umsetzung, die Lösung der erfindungsgemäß eingesetzten Homogenkatalysatoren.

Zur Herstellung der Endprodukte n-Butyraldehyd und n-Butanol wird der Reaktionsmischung Wasser in einem Molverhältnis, bezogen auf das dem Reaktor zugeführte Addukt II, von im allgemeinen 1 : 1 bis 100 : 1, vorzugsweise 2 : 1 bis 20 : 1 und besonders bevorzugt von 3 : 1 bis 10 : 1 zugesetzt. Bei der diskontinuierlichen Betriebsweise kann das Wasser gemeinsam mit den anderen Reaktanten, dem Addukt II und dem Homogenkatalysator, im Reaktor vorgelegt werden, es kann aber auch vorteilhaft sein, das Wasser erst nach dem Beginn der Umsetzung in den Reaktor zu dosieren. Welche dieser Verfahrensweisen letztlich gewählt wird, hängt vom jeweils verwendeten Katalysator und den angewandten Druck- und Temperaturbedingungen ab. Zweckmäßigerweise wird die optimale Verfahrensweise für den jeweils verwendeten Katalysator in einem Vorversuch ermittelt. In analoger Weise kann bei der kontinuierlichen Ausgestaltung des Verfahrens, z.B. in einem Rohr- oder Kaskadenreaktor, das Wasser gemeinsam mit den übrigen Reaktanten in den Reaktor geleitet oder aber erst nach einer gewissen Verweilzeit der Reaktanten im Reaktor, über einen separaten Einlaß in den Reaktor dosiert werden.

Ist das gewünschte Endprodukt n-Butanol, wird der Reaktionsmischung, zusätzlich zu dem für die Hydrolyse des Enamins IV benötigten Wasser noch Wasserstoff in einem Molverhältnis, bezogen auf das dem Reaktor zugeführte Addukt II, von im allgemeinen 1 : 1 bis 100 : 1, vorzugsweise von 1 : 1 bis 10 : 1 und besonders bevorzugt von 1 : 1 bis 3 : 1 zugemischt. Diese Zumischung kann bei diskontinuierlicher Betriebsweise des Verfahrens durch Einpressen der erforderlichen Wasserstoffmenge in den Reaktor oder durch Dispergieren des Wasserstoffs im Reaktionsmedium, beispielsweise mittels Blasensäulen oder mittels mit Strahldüsen zur Dispergierung des Wasserstoffs ausgestatteten Schlaufenreaktoren erfolgen. Die Zumischung des Wasserstoffs kann bei der Beschickung des Reaktors mit den übrigen Reaktanten, also dem Addukt II, dem Wasser und dem Homogenkatalysator, erfolgen, der Wasserstoff kann aber auch vorteilhaft nachträglich, nach dem Beginn der Umsetzung, in die Reaktionsapparatur eingespeist werden. Welche dieser Verfahrensweisen letztlich gewählt wird, hängt vom verwendeten Katalysator und den jeweils angewandten Druck- und Temperaturbedingungen sowie von der Konstruktion des Reaktors ab. Zweckmäßigerweise wird die optimale Verfahrensweise in einem Vorversuch ermittelt. In analoger Weise kann bei der kontinuierlichen Ausgestaltung des Verfahrens, z.B. in einem Rohrreaktor, einem Blasensäulenreaktor oder einer Füllkörperkolonne, der Wasserstoff gemeinsam mit den übrigen Reaktanten in den Reaktor eingebracht oder aber erst nach einer gewissen Verweilzeit der Reaktanten im Reaktor, über einen separaten Einlaß in diesen eingeleitet werden.

Ist das gewünschte Endprodukt eine Mischung aus n-Butanol und n-Butyraldehyd, so kann das Mengenverhältnis dieser Produkte in der Produktmischung beispielsweise über den Zusatz des Wasserstoffs und/oder die angewandte Reaktionstemperatur eingestellt werden. Werden unterstöchiometrische Mengen an Wasserstoff eingesetzt, wird selbstverständlich nur ein Teil des Ausgangsmaterials zu n-Butanol hydriert und durch Anwendung einer niedrigeren Reaktionstemperatur kann die Geschwindigkeit der Hydrierreaktion so weit verlangsamt werden, daß nur ein Teil des Ausgangsmaterials zu n-Butanol hydriert wird.

Die Durchführung der Teilreaktionen c) und d) in mindestens zwei Verfahrensstufen mit Hilfe der genannten Homogenkatalysatoren kann diskontinuierlich, z.B. in Rührkesseln oder kontinuierlich, z.B. in Rührkesseln und/oder Rohrreaktoren durchgeführt werden.

Die Isomerisierung des Addukts II zum Enamin IV in der ersten Stufe kann in An- oder Abwesenheit von zugesetzten Lösungsmitteln, wie aliphatischen oder aromatischen Kohlenwasserstoffen, z.B. Toluol, Benzol oder Cyclohexan, Alkoholen, z.B. Butanole, insbesondere n-Butanol, höhere Fettalkohole oder Glykole, Ethern, z.B. Dibutylether, Tetrahydrofuran, Dioxan oder niedermolekulare Polyalkylenglykole, halogenierten aliphatischen oder aromatischen Kohlenwasserstoffen, z.B. Chloroform, Dichlormethan, Chlorbenzol, Dichlorbenzol, Sulfoxiden oder Sulfonen, z.B. Dimethylsulfoxid oder Sulfolan erfolgen.

Statt in diesen herkömmlichen Lösungsmitteln kann die Isomerisierung des Addukts II zum Enamin IV auch in einer Phosphin-Schmelze erfolgen. Vorteilhaft kann diese Verfahrensweise bei Phosphin-haltigen Homogenkatalysatoren angewandt werden. Das dabei als Lösungsmittel dienende Phosphin kann im allgemeinen beliebig gewählt werden, vorzugsweise wird jedoch dasjenige Phosphin in der Schmelze verwendet, das im als Homogenkatalysator dienenden Übergangsmetallelementkomplex als Ligand dient.

In der zweiten Stufe kann dann mit einem bei der einstufigen Verfahrensweise beschriebenen Homogen-Katalysator die Hydrolyse des Enamins IV zu Butyraldehyd oder die kombinierte Hydrolyse/Hydrierung zu n-Butanol erfolgen.

Ist das gewünschte Produkt n-Butyraldehyd und/oder n-Butanol kann wie bei dem einstufigen Prozeß beschrieben vorgegangen werden.

Als Säuren für die Hydrolyse des Enamins IV zu Butyraldehyd können beispielsweise herkömmliche, nicht oxidierende Brönsted-Säuren, wie Halogenwasserstoffsäuren, z.B. Salzsäure, Schwefelsäure, Phosphorsäure, Perchlorsäure, Flußsäure, Tetrafluoroborsäure, Methansulfonsäure und Toluolsulfonsäure oder organische Säuren wie Ameisensäure, Essigsäure, Propionsäure oder Disäuren wie Oxalsäure Verwendung finden, vorzugsweise werden aber feste Brönsted-Säuren, insbesondere organische oder anorganische Kationentauscher oder Essig- oder Oxalsäure eingesetzt.

Da die optimalen Mengen der zu verwendenden Säuren je nach Säure stark schwanken, muß der Fachmann die jeweilige Menge in einem Vorversuch in an sich bekannter Weise ermitteln.

Unter organischen Kationenaustauschern werden pulverförmige, gelartige oder makroporöse, polymere Polyelektrolyte verstanden, die Brönsted-acide funktionelle Gruppe, wie Sulfonsäure-, Phosphonsäure- oder Carboxylgruppen auf einer polymeren Matrix tragen, beispielsweise sulfonierte Phenol-Formaldehyd-Harze, sulfonierte Styrol-Divinylbenzol-Copolymere, sulfoniertes Polystyrol, Poly(perfluoralkylen)sulfonsäuren oder sulfonierte Kohlen. Im erfindungsgemäßen Verfahren können diese Kationenaustauscher in Form handelsüblicher Produkte eingesetzt werden, wie sie beispielsweise unter den Handelsnamen Amberlite®, Dowex®, Amberlyst®, Lewatit®, Wofatit®, Permutit® und Nafion® im Handel erhältlich sind. Zweckmäßigerweise kommen die Kationenaustauscher im erfindungsgemäßen Verfahren in ihrer protonierten Form, der sogenannten H⁺-Form zur Anwendung. Als geeignete organische Kationenaustauscher seien beispielhaft die Handelsprodukte Amberlite® 200, Amberlite® IR 120, Amberlite® IR 132 E, Lewatit® SC 102, Lewatit® SC 104, Lewatit® SC 108, Lewatit® SPC 108, Lewatit® SPC 112, Lewatit® SPC 118 und Amberlyst® 15 genannt.

Anstelle organischer, saurer Kationenaustauscher können im erfindungsgemäßen Verfahren auch feste, Brönsted-acide wirkende anorganische Feststoffe eingesetzt werden, beispielsweise Zeolithe, wie β-Zeolithe oder Y-Zeolithe in der H⁺-Form, Bleicherden, wie Bentonite, Montmorillonite oder Attapulgite, nichtzeolithische Molekularsiebe auf Phosphatbasis, wie sie beispielsweise Gegenstand von US-A 4 440 871, US-A 4 310 440, US-A 4 567 029, US-A 4 554 143, US-A 4 500 651, EP-A 158 976, EP-A 158 349, EP-A 159 624 sind, sowie saure oder säureimprägnierte Metalloxide, deren Herstellung z.B. in US-A 4 873 017 beschrieben ist. Bevorzugte Brönsted-acide anorganische Feststoffe sind β-Zeolithe oder Y-Zeolithe in der H⁺-Form, insbesondere β-Zeolithe in der H⁺-Form. β-Zeolithe sind z.B. nach dem Verfahren von US-A 4 891 458 erhältlich.

Werden in dieser Teilreaktion des erfindungsgemäßen Verfahrens flüssige oder gelöste Brönsted-Säure-Katalysatoren, insbesondere Essigsäure oder Oxalsäure eingesetzt, wird im allgemeinen so vorgegangen, daß man das Enamin IV und Wasser in flüssiger Form in die vorgelegte Säure einleitet und die gebildeten Produkte destillativ oder durch Strippen aus der Reaktionszone entfernt. Hierzu können an sich übliche Reaktoren wie Blasensäulen, Schlaufenreaktoren u.ä. verwendet werden. Vorteilhaft kann das Gemisch z.B. mittels Strahldüsen in die Säure eingebracht werden. Die Produkte können von der wäßrigen Lösung der Brönsted-Säure auch mittels Phasenabscheidern abgetrennt werden. Anstelle von Blasensäulen oder Schlaufenreaktoren können auch Rührkesselkaskaden verwendet werden. Werden im erfindungsgemäßen Verfahren jedoch feste Brönsted-Säuren in Form der obengenannten organischen oder anorganischen Katalysatoren verwendet, insbesondere organische Ionenaustauscher, werden diese vorzugsweise in einem Festbett angeordnet und von der flüssigen Reaktionsmischung in der Sumpf- oder Rieselfahrweise durchströmt. Das Katalysatorfestbett kann z.B. in Rohrreaktoren oder vorzugsweise in Reaktorkaskaden eingebaut werden.

Nach beendeter Umsetzung wird das Reaktionsprodukt im allgemeinen destillativ aufgearbeitet, wobei der verwendete Homogenkatalysator aus dem Sumpf der Destillation zurückgewonnen und gewünschtenfalls erneut verwendet werden kann, beispielsweise durch Rückführung der Katalysatorlösung in die Verfahrensstufe der Isomerisierung des Addukts II zum Enamin IV und dessen Hydrolyse und Hydrierung. Ist die Rückführung des Katalysators im erfindungsgemäßen Verfahren erwünscht, kann dem Reaktionsgemisch zweckmäßigerweise noch ein Lösungsmittel, vorzugsweise ein Lösungsmittel, das bei einer höheren Temperatur als die Reaktionsprodukte n-Butanol und n-Butyraldehyd siedet, zugefügt werden. Ist der verwendete Homogenkatalysator unter den Bedingungen der Destillation chemisch und thermisch stabil, kann vom Zusatz eines hochsiedenden Lösungsmittels abgesehen und der Homogenkatalysator in fester Form wieder in die Umsetzung zurückgeführt werden. Bei der destillativen Aufarbeitung wird weiterhin das Reaktionsprodukt n-Butyraldehyd und/oder n-Butanol von dem in der vorausgegangenen Verfahrensstufe aus dem Enamin IV durch Hydrolyse oder Hydrierung freigesetzte Amin HNR¹R² I abgetrennt, das wiederum in die erste Verfahrensstufe des erfindungsgemäßen Verfahrens, die Addition des Amins I an 1,3-Butadien, zurückgeführt wird. Als wertvolle Nebenprodukte des erfindungsgemäßen Verfahrens können bei der destillativen Aufarbeitung des Reaktionsproduktes, die als Folge der teilweisen Dimerisierung des Butadiens entstandenen Octanole bzw. die diesen Alkoholen entsprechenden Aldehyde, gewonnen werden.

In einer weiteren Ausgestaltung des erfindungsgemäßen Verfahrens wird die Isomerisierung des Addukts II zum Enamin IV und dessen Hydrolyse oder Hydrierung zu n-Butyraldehyd und/oder n-Butanol in einer einzigen Verfahrensstufe unter Verwendung eines heterogenen Katalysators ausgeführt, wobei das Verfahren wahlweise in flüssiger Phase oder in der Gasphase durchgeführt werden kann.

Es wurde überraschenderweise gefunden, daß als Katalysatoren sowohl für die Isomerisierung des Addukts II zum Enamin IV als auch für die Hydrolyse des Enamins IV zu n-Butyraldehyd oder für die kombinierte Hydrolyse/Hydrierung des Enamins IV zu n-Butanol an sich gängige heterogene Hydrierkatalysatoren, die im Reaktionsmedium im wesentlichen unlöslich sind, verwendet werden können. Von diesen Hydrierkatalysatoren sind solche bevorzugt, die ein oder mehrere Elemente der Gruppe Ib, VIb, VIIb und VIIIb, gegebenenfalls in Kombination mit einem oder mehreren Elementen der Gruppe Vb, des Periodensystems der Elemente, insbesondere Kupfer, Chrom, Molybdän, Wolfram, Rhenium, Ruthenium, Kobalt, Nickel, Rhodium, Iridium, Palladium und/oder Platin, gegebenenfalls in Kombination mit Eisen, enthalten.

Besonders aktive Hydrierkatalysatoren wie Nickel oder die Platinmetalle werden vorteilhaft mit Hauptgruppenelementen, die als Katalysatorgift wirken, dotiert und auf diese Weise partiell vergiftet. Durch diese Maßnahme kann eine höhere Selektivität bei der kombinierten Hydrolyse/Hydrierung des Enamins IV zu n-Butanol erzielt werden. Geeignete Hauptgruppenelemente sind z.B. die Chalcogene, wie Schwefel, Selen und Tellur, sowie die Elemente Phosphor, Arsen, Antimon, Wismut, Zinn, Blei und Thallium.

Im erfindungsgemäßen Verfahren können als Heterogenkatalysatoren z.B. sogenannte Fällungskatalysatoren verwendet werden. Solche Katalysatoren können hergestellt werden, indem man ihre katalytisch aktiven Komponenten aus deren Salzlösungen, insbesondere aus den Lösungen von deren Nitraten und/oder Acetaten, beispielsweise durch Zugabe von Lösungen von Alkalimetall- und/oder Erdalkalimetallhydroxid- und/oder-Carbonat-Lösungen, als z.B. schwerlösliche Hydroxyde, Oxidhydrate, basische Salze oder Carbonate ausfällt, die erhaltenen Niederschläge anschließend trocknet und diese dann durch Calcinierung bei im allgemeinen 300 bis 700°C, insbesondere 400 bis 600°C in die betreffenden Oxide, Mischoxide und/oder gemischtvalentigen Oxide umwandelt, welche z.B. durch eine Behandlung mit Reduktionsmitteln, wie Wasserstoff oder Wasserstoff enthaltenden Gasen, bei in der Regel 20 bis 700°C, insbesondere bei 20 bis 300°C, zu den betreffenden Metallen und/oder zu oxidischen Verbindungen niedriger Oxidationsstufe reduziert und in die eigentliche, katalytisch aktive Form überführt werden. Dabei wird in der Regel solange reduziert, bis kein Wasser mehr gebildet wird. Bei der Herstellung von Fällungskatalysatoren, die ein Trägermaterial enthalten, kann die Fällung der katalytisch aktiven Komponenten in Gegenwart des betreffenden Trägermaterials erfolgen. Die katalytisch aktiven Komponenten können vorteilhaft aber auch gleichzeitig mit dem Trägermaterial aus den betreffenden Salzlösungen gefällt werden.

Bevorzugt werden im erfindungsgemäßen Verfahren Hydrierkatalysatoren eingesetzt, welche die die Hydrierung katalysierenden Metalle oder Metallverbindungen auf einem Trägermaterial abgeschieden enthalten. Außer den obengenannten Fällungskatalysatoren, welche außer den katalytisch aktiven Komponenten noch zusätzlich ein Trägermaterial enthalten, eignen sich für das erfindungsgemäße Verfahren im allgemeinen solche Trägerkatalysatoren, bei denen die katalytisch wirkenden Komponenten z.B. durch Imprägnierung auf ein Trägermaterial aufgebracht worden sind.

Die Art der Aufbringung der katalytisch aktiven Metalle auf den Träger ist in der Regel nicht kritisch und kann auf verschiedenerlei Art und Weise bewerkstelligt werden. Die katalytisch aktiven Metalle können auf diese Trägermaterialien z.B. durch Tränkung mit Lösungen oder Suspensionen der Salze oder Oxide betreffenden Elemente, Trocknung und anschließende Reduktion der Metallverbindungen zu den betreffenden Metallen oder Verbindungen niederer Oxidationsstufe mittels eines Reduktionsmittels, vorzugsweise mit Hilfe von Wasserstoff, Wasserstoff enthaltenden Gasen oder Hydrazin, aufgebracht werden. Eine andere Möglichkeit zur Aufbringung der katalytisch aktiven Metalle auf diese Träger besteht darin, die Träger mit Lösungen thermisch leicht zersetzbarer Salze, z.B. mit Nitraten oder mit thermisch leicht zersetzbaren Komplexverbindungen, z.B. Carbonyl- oder Hydrido-Komplexen der katalytisch aktiven Metalle, zu imprägnieren und den so getränkten Träger zwecks thermischer Zersetzung der adsorbierten Metallverbindungen auf Temperaturen von 300 bis 600°C zu erhitzen. Diese thermische Zersetzung wird vorzugsweise unter einer Schutzgasatmosphäre vorgenommen. Geeignete Schutzgase sind z.B. Stickstoff, Kohlendioxid, Wasserstoff oder die Edelgase. Weiterhin können die katalytisch aktiven Metalle auf dem Katalysatorträger durch Aufdampfung oder durch Flammspritzen abgeschieden werden.

Der Gehalt dieser Trägerkatalysatoren an den katalytisch aktiven Metallen ist prinzipiell für das Gelingen des erfindungsgemäßen Verfahrens nicht kritisch. Es versteht sich für den Fachmann von selbst, daß höhere Gehalte dieser Trägerkatalysatoren an katalytisch aktiven Metallen zu höheren Raum-Zeit-Umsätzen führen als niedrigere Gehalte. Im allgemeinen werden aber Trägerkatalysatoren verwendet, deren Gehalt an katalytisch aktiven Metallen 0,1 bis 80 Gew.-%, vorzugsweise 0,5 bis 30 Gew.-%, bezogen auf den gesamten Katalysator beträgt. Da sich diese Gehaltsangaben auf den gesamten Katalysator inklusive Trägermaterial beziehen, die unterschiedlichen Trägermaterialien jedoch sehr unterschiedliche spezifische Gewichte und spezifische Oberflächen haben, können diese Angaben aber auch unter- oder überschritten werden, ohne daß sich dies nachteilig auf das Ergebnis des erfindungsgemäßen Verfahrens auswirkt. Selbstverständlich können auch mehrere der katalytisch aktiven Metalle auf dem jeweiligen Trägermaterial aufgebracht sein. Weiterhin können die katalytisch aktiven Metalle beispielsweise nach den Verfahren von DE-A 25 19 817, EP-A 147 219 und EP-A 285 420 auf den Träger aufgebracht werden. In den Katalysatoren gemäß den vorgenannten Schriften liegen die katalytisch aktiven Metalle als Legierung vor, die durch thermische Behandlung und/oder Reduktion der z.B. durch Tränkung auf einem Träger abgeschiedenen Salze oder Komplexe der zuvor genannten Metalle, erzeugt werden.

Die Aktivierung der Fällungskatalysatoren als auch der Trägerkatalysatoren kann auch in situ in der Reaktionsmischung durch den dort anwesenden Wasserstoff erfolgen, bevorzugt werden diese Katalysatoren jedoch vor ihrer Verwendung im erfindungsgemäßen Verfahren aktiviert.

Als Trägermaterialien können im allgemeinen die Oxide des Aluminiums und Titans, Zirkoniumdioxid, Siliziumdioxid, Kieselgur, Kieselgel, Tonerden, z.B. Montmorillonite, Silikate, wie Magnesium- oder Aluminiumsilikate, Zeolithe, wie ZSM-5 oder ZSM-10-Zeolithe sowie Aktivkohle verwendet werden. Bevorzugte Trägermaterialien sind Aluminiumoxide, Titandioxide, Zirkoniumdioxid und Aktivkohle. Selbstverständlich können auch Mischungen verschiedener Trägermaterialien als Träger für im erfindungsgemäßen Verfahren anwendbare Katalysatoren dienen.

Als für die Durchführung der Teilreaktionen c) und d) in einer einzigen Verfahrensstufe einsetzbare Heterogenkatalysatoren seien die folgenden Katalysatoren beispielhaft genannt: Platindioxid, Palladium auf Aluminiumoxid, Palladium auf Siliziumdioxid, Palladium auf Bariumsulfat, Rhodium auf Aktivkohle, Rhodium auf Aluminiumoxid, Ruthenium auf Siliziumdioxid oder Aktivkohle, Nickel auf Siliziumdioxid, Kobalt auf Siliziumdioxid, Kobalt auf Aluminiumoxid, Carbonyleisenpulver, Rheniumschwarz, Raney-Rhenium, Rhenium auf Aktivkohle, Rhenium-Palladium auf Aktivkohle, Rhenium-Platin auf Aktivkohle, Kupfer auf Kieselgur, Kupfer auf Kieselgel, Kupfer auf Titandioxid, Kupfer auf Zirkoniumdioxid, Kupfer auf Magnesiumsilikat, Kupfer auf Aluminiumsilikat, Kupfer auf Montmorillonit, Kupfer auf Zeolith, Raney-Kupfer, Platinoxid-Rhodiumoxid-Mischungen, Platin-Palladium auf Aktivkohle, Kupferchromit, Bariumchromit, Nickel-Chromoxid auf Aluminiumoxid, Dirheniumheptoxid (Re₂O₇), Kobaltsulfid, Nickelsulfid, Molybdän(VI)sulfid, Kupfer-Molybdän(VI)oxid-Siliziumdioxid-Aluminiumoxid-Katalysatoren, mit Selen oder Blei partiell vergiftete Palladium-auf-Aktivkohlekatalysatoren sowie die Katalysatoren gemäß DE-A 39 32 332, US-A 3 449 445, EP-A 44 444, EP-A 147 219, DE-A 39 04 083, DE-A 23 21 101, EP-A 415 202, DE-A 23 66 264 und EP-A 100 406.

Vorteilhaft können im erfindungsgemäßen Verfahren auch Hydrierkatalysatoren verwendet werden, die Brönsted- und/oder Lewis-acide-Zentren enthalten. Bei der Verwendung derartiger Katalysatoren ist im allgemeinen die zusätzliche Zugabe einer Brönsted- oder Lewis-Säure zur Reaktionsmischung nicht erforderlich.

Als Brönsted- oder Lewis-saure Zentren können z.B. die katalytisch aktiven Metalle selber wirken, wenn diese bei der Aktivierung des Katalysators mit Wasserstoff oder Wasserstoff enthaltenden Gasen nicht vollständig zu den betreffenden Metallen reduziert werden. Dies gilt z.B. für die rhenium- und chromithaltigen Katalysatoren, wie Rheniumträgerkatalysatoren liegt das Rhenium als Mischung von Rheniummetall mit Rheniumverbindungen in höheren Oxidationsstufen vor, wobei letztere Wirkungen wie Lewis- oder Brönsted-Säuren entfalten können. Weiterhin können solche Lewis- oder Brönsted-aciden Zentren über das verwendete Trägermaterial in den Katalysator eingebracht werden. Als Lewis- oder Brönsted-acide Zentren enthaltende Trägermaterialien seien z.B. Zirkoniumdioxid, Siliziumdioxid, die Silikate, Tonerden, Zeolithe und Aktivkohle genannt.

Besonders bevorzugt werden deshalb im erfindungsgemäßen Verfahren als Hydrierkatalysatoren Trägerkatalysatoren verwendet, die Elemente der I., VI., VII. und/oder VIII. Nebengruppe des Periodensystems der Elemente, insbesondere der Elemente der I., VII. und VIII. Nebengruppe des Periodensystems der Elemente auf einem Brönsted- oder Lewis-acide wirkenden Trägermaterial abgeschieden enthalten. Besonders vorteilhafte Katalysatoren sind z.B. Rhenium auf Aktivkohle, Rhenium auf Zirkoniumdioxid, Rhenium auf Titandioxid, Rhenium auf Siliziumdioxid, Kupfer auf Aktivkohle, Kupfer aus Siliziumdioxid, Kupfer auf Kieselgur, Kupfer auf Kieselgel, Kupfer auf Titandioxid, Kupfer auf Zirkoniumdioxid, Kupfer auf Magnesiumsilikat, Kupfer auf Aluminiumsilikat, Kupfer auf Bleicherde, Kupfer auf Zeolith, Ruthenium auf Aktivkohle, Ruthenium auf Aluminiumoxid, Ruthenium auf Siliziumdioxid, Ruthenium auf Titandioxid und mit Selen oder Blei partiell vergiftete Palladium auf Aktivkohle-Katalysatoren.

Hydrierkatalysatoren, welche selbst keine derartigen Brönsted- oder Lewis-aciden Zentren haben, können Lewis- oder Brönsted-acide-Komponenten, wie Zeolithe, Aluminium- oder Siliziumoxide, Phosphorsäure oder Schwefelsäure, zugesetzt werden. Sie werden im allgemeinen in Mengen von 0,01 bis 5 Gew.-%, vorzugsweise von 0,05 bis 0,5 Gew.-% und besonders bevorzugt von 0,1 bis 0,4 Gew.-%, bezogen auf das Gewicht des eingesetzten Katalysators, zugesetzt.

Weiterhin sind zur Isomerisierung des Addukts II zum Enamin IV und dessen Hydrolyse oder Hydrierung zu n-Butyraldehyd und/oder n-Butanol in einer einzigen Verfahrensstufe Heterogenkatalysatoren geeignet, welche die zur homogenen Katalyse dieser Verfahrensstufe verwendbaren Komplexverbindungen von Übergangsmetallelementen aus der Gruppe VIb und VIIIb des Periodensystems der Elemente in heterogenisierter Form enthalten, beispielsweise solche, in denen das betreffende Übergangsmetallelement an eine polymere Matrix fixiert ist.

Solche polymeren Matrizes können Harze, wie Styrol-Divinylbenzol-Harze (US 37 25 489) oder Phenol-Formaldehyd-Harze sein, an die die betreffenden zur Komplexierung des Übergangsmetallelements dienenden Liganden vorzugsweise kovalent gebunden sind, welche wiederum mit den betreffenden Übergangsmetallen Komplexe bilden und diese derart quasi immobilisieren. Derartige heterogenisierte, polymer gebundene Übergangsmetallkomplex-Katalysatoren mit 2,2'-Bipyridin- oder 1,10-Phenanthrolin-Liganden oder heterogenisierte Phosphin- oder Phosphitkomplexe der katalytisch aktiven Übergangsmetallelemente können z.B. nach den bei der Erläuterung von Teilreaktion a) für die Herstellung dieser Katalysatoren genannten Literaturverfahren hergestellt werden. Organotrioxorhenium(VII)-Katalysatoren können z.B. nach dem Verfahren von DE-A 39 02 357 an stickstoffhaltige Polymere, wie Polyvinylpyrrolidon, Poly(2-vinylpyridin), (2-Vinylpyridin-)Styrol-Copolymere, Polyacrylsäureamide, Polyimide, Polyamide und Polyurethane durch koordinative Bindung fixiert und auf diese Weise heterogenisiert, als Heterogenkatalysator erfindungsgemäß verwendet werden.

Mit den genannten Heterogenkatalysatoren kann die Isomerisierung des Addukts II zum Enamin IV und dessen Hydrolyse oder Hydrierung zu n-Butyraldehyd und/oder n-Butanol in einer einzigen Verfahrensstufe sowohl kontinuierlich als auch diskontinuierlich durchgeführt werden.

Wird diese Umsetzung in flüssiger Phase durchgeführt, so kann der Heterogenkatalysator sowohl im flüssigen Reaktionsmedium suspendiert oder vorzugsweise in einem Festbett oder mehreren Festbetten angeordnet, eingesetzt werden. Das Verfahren kann bei Einsatz eines im flüssigen Reaktionsmedium suspendierten Heterogenkatalysators z.B. in Rührkesseln oder Schlaufenreaktoren durchgeführt werden. Bei Anwendung eines in einem Festbett angeordneten Heterogenkatalysators wird das Reaktionsgut im allgemeinen in der Sumpf- oder Rieselfahrweise über das Katalysatorfestbett geleitet.

Sowohl die Hydrierung des Enamins IV als auch dessen Hydrolyse oder Hydrierung können in adiabatisch oder isotherm betriebenen Reaktoren vorgenommen werden. Im allgemeinen wird hierbei der Katalysator mit der flüssigen Reaktionsmischung mit einer Raumgeschwindigkeit von 0,01 bis 10, vorzugsweise von 0,05 bis 3 und besonders bevorzugt von 0,08 bis 1 kg Amin/l Katalysator · h belastet. Bei Anwendung der Heterogenkatalysatoren kann die Umsetzung in An- oder Abwesenheit eines Lösungsmittels erfolgen. Als Lösungsmittel können die gleichen Lösungsmittel Anwendung finden, welche auch bei der Durchführung des Verfahrens unter homogener Katalyse verwendet werden können.

Wie bei der Durchführung der Teilreaktionen c) und d) des erfindungsgemäßen Verfahrens unter homogener Katalyse bereits beschrieben wurde, kann das zur Herstellung der Endprodukte n-Butyraldehyd und/oder n-Butanol benötigte Wasser entweder gemeinsam mit dem Addukt II dem Reaktor zugeführt und/oder über separate Zuleitungen, in einen oder in mehrere Teilströme aufgeteilt, an verschiedenen Stellen in die Katalysatorschüttung eingespeist werden. Gleiches gilt für die Zufuhr von Wasser und Wasserstoff bei der Herstellung des Endproduktes n-Butanol.

Das zur Herstellung von n-Butyraldehyd benötigte Wasser wird bei Durchführung des Verfahrens unter heterogener Katalyse bezüglich des dem Reaktor zugeführten Addukts II in einem Molverhältnis von im allgemeinen 1 bis 100, vorzugsweise von 1 bis 50 und besonders bevorzugt von 1 bis 10 zugesetzt. Die kombinierte Isomerisierung des Addukts II zum Enamin IV und dessen Hydrolyse zu n-Butyraldehyd in einer einzigen Verfahrensstufe am Heterogenkatalysator in flüssiger Phase wird im allgemeinen bei einer Temperatur von 20 bis 400°C, vorzugsweise von 30 bis 300°C und besonders bevorzugt von 80 bis 200°C und bei einem Druck von im allgemeinen 1 bis 300 bar, vorzugsweise von 2 bis 150 bar, insbesondere von 5 bis 100 bar durchgeführt.

Der zur Herstellung von n-Butanol zusätzlich zum Wasser benötigte Wasserstoff wird bei der Durchführung des Verfahrens unter heterogener Katalyse bezüglich des dem Reaktor zugeführten Addukts II in einem Molverhältnis von im allgemeinen 1 bis 100, vorzugsweise von 1,5 bis 80, insbesondere von 2 bis 40 zugesetzt. Die kombinierte Isomerisierung des Addukts II zum Enamin IV und dessen Hydrolyse/Hydrierung zu n-Butanol in einer einzigen Verfahrensstufe am Heterogenkatalysator in flüssiger Phase wird im allgemeinen bei einer Temperatur von 20 bis 400°C, vorzugsweise von 30 bis 300°C und besonders bevorzugt von 80 bis 200°C und bei einem Druck von im allgemeinen 1 bis 300 bar, vorzugsweise von 5 bis 250 bar, insbesondere von 20 bis 200 bar ausgeführt. Es versteht sich von selbst, daß die zur Herstellung von n-Butanol aus dem Addukt II benötigte Wassermenge gleich der zur Herstellung von n-Butyraldehyd aus dem Addukt II benötigten Wassermenge ist.

Ist das gewünschte Endprodukt eine Mischung aus n-Butyraldehyd und n-Butanol, werden im allgemeinen Wasser und Wasserstoff bezüglich des in den Reaktor eingebrachten Addukts II in analoger Weise wie zuvor beschrieben, in einem Mengenverhältnis zugemischt, das die Gewinnung beider Endprodukte im gewünschten Produktverhältnis ermöglicht. Darüber hinaus läßt sich das Produktverhältnis dieser beiden Endprodukte im Reaktoraustrag auch über die Verwendung bestimmter Heterogenkatalysatoren steuern, die über eine hohe Hydrolyseaktivität und eine verglichen damit, relativ geringe Hydrieraktivität verfügen. Zu diesem Zweck können z.B. bezüglich ihrer Hydriereigenschaften inaktivierte oder partiell vergiftete Katalysatoren, z.B. mit Selen oder Blei partiell vergiftete Palladium auf Aktivkohle-Katalysatoren vorteilhaft angewandt werden.

Der flüssige Reaktionsaustrag aus dieser Verfahrensstufe wird im allgemeinen destillativ, in analoger Weise, wie es für die Durchführung dieser Verfahrensstufe mit Homogenkatalysatoren bereits beschrieben wurde, aufgearbeitet. Naturgemäß entfällt bei der Anwendung von Heterogenkatalysatoren die Rückführung des Katalysators, wie sie unter Umständen bei Anwendung von Homogenkatalysatoren zweckmäßig und vorteilhaft ist. Die Rückführung des in dieser Verfahrensstufe wieder freigesetzten Amins R¹R²NH I in die Verfahrensstufe der Addition des Amins R¹R²NH I an 1,3-Butadien kann vorteilhaft auf analoge Weise, wie für die Umsetzung dieser Verfahrensstufe mit Homogenkatalysatoren bereits beschrieben, geschehen.

Wie bereits erwähnt, kann die Isomerisierung des Addukts II zum Enamin IV und dessen Hydrolyse oder Hydrierung zu n-Butyraldehyd und/oder n-Butanol in einer einzigen Verfahrensstufe vorteilhaft in der Gasphase durchgeführt werden. Hierzu können an sich übliche Reaktoren für Gasphasenreaktionen verwendet werden, beispielsweise solche, in denen der Katalysator in einem Festbett oder in einer Wirbelschicht angeordnet ist. Die Reaktoren können adiabatisch oder isotherm betrieben werden. Bei Anwendung einer Festbettanordnung des Katalysators kann der Katalysator in einem oder vorteilhaft zwecks Verbesserung der Abführung der Reaktionswärme in mehreren, beispielsweise 2 bis 10, vorzugsweise 2 bis 5 Festbetten, angeordnet sein. Bei Verwendung mehrerer Katalysatorfestbetten oder bei adiabatischer Betriebsweise des Reaktors kann es vorteilhaft sein, durch eine Zwischenbettkühlung des Reaktionsgases und/oder durch Eindüsen zusätzlicher Mengen an kühlen Reaktanten wie Wasserstoff, Wasser, Addukt II oder Enamin IV zwischen den einzelnen Festbetten, die Temperatur des Reaktionsgases nach Verlassen des vorhergehenden Festbetts und vor Eintritt in das nachfolgende Festbett abzusenken, um die Selektivität der Umsetzung zu erhöhen. Die Abführung der Reaktionswärme ist auch durch eine Kreisgasfahrweise möglich. Zweckmäßigerweise wird bei Anwendung mehrerer Festbette in den einzelnen Festbetten vor dem letzten Festbett die Umsetzung nur bis zu einem Teilumsatz, beispielsweise bis zu einem Umsatz von 50 bis 98 %, durchgeführt. Die Reaktionsgase können gewünschtenfalls mit einem unter den Reaktionsbedingungen inerten Gas, wie Stickstoff, gesättigten Kohlenwasserstoffen oder Argon, verdünnt werden.

Das zur Herstellung des Endprodukts n-Butyraldehyd benötigte Wasser wird bei der Durchführung des Verfahrens in der Gasphase bezüglich des dem Reaktor zugeführten Addukts II in einem Molverhältnis von im allgemeinen 1 bis 100, vorzugsweise von 1 bis 50 und besonders bevorzugt von 1 bis 10 zugesetzt. Das Wasser kann dem Reaktor gemeinsam mit dem Addukt II und/oder, wie oben dargelegt, in mehrere Teilströme aufgeteilt an unterschiedlichen Stellen des Reaktors zugeführt werden. Im allgemeinen wird der Katalysator mit dem Reaktionsgas, enthaltend im wesentlichen das Addukt II, Wasser und gewünschtenfalls ein Inertgas, mit einer Raumgeschwindigkeit von 0,01 bis 10, vorzugsweise von 0,05 bis 3 und besonders bevorzugt von 0,07 bis 1 kg Reaktionsgas/l Katalysator · h belastet. Die Umsetzung, umfassend die Isomerisierung des Addukts II zum Enamin IV und dessen Hydrolyse, wird im allgemeinen bei einer Temperatur von 70 bis 400°C, vorzugsweise von 90 bis 350°C und besonders bevorzugt von 110 bis 230°C und bei einem Druck von im allgemeinen 0,5 bis 100 bar, vorzugsweise von 0,8 bis 20 bar und besonders bevorzugt 1 bis 10 bar ausgeführt.

Der zur Herstellung des Endprodukts n-Butanol zusätzlich zum Wasser benötigte Wasserstoff wird bei der Durchführung des Verfahrens in der Gasphase bezüglich des dem Reaktor zugeführten Addukts II in einem Molverhältnis von im allgemeinen 1 bis 200, vorzugsweise von 1,5 bis 80 und besonders bevorzugt von 2 bis 30 zugesetzt. Wasserstoff kann dem Reaktor gemeinsam mit dem Addukt II und/oder, wie oben dargelegt, in mehrere Teilströme aufgeteilt, an verschiedenen Stellen des Reaktors zugeführt werden. Im allgemeinen wird der Katalysator mit dem Reaktionsgas, enthaltend im wesentlichen das Addukt II, Wasser, Wasserstoff und gewünschtenfalls ein Inertgas, mit einer Raumgeschwindigkeit von 0,01 bis 10, vorzugsweise von 0,05 bis 3, insbesondere von 0,07 bis 1 kg Reaktionsgas/l Katalysator · h belastet. Die Umsetzung, umfassend die Isomerisierung des Addukts II zum Enamin IV und dessen kombinierte Hydrolyse/Hydrierung, wird im allgemeinen bei Temperaturen von 20 bis 400°C, vorzugsweise von 100 bis 350°C und besonders bevorzugt von 150 bis 250°C und bei einem Druck von im allgemeinen 0,5 bis 100 bar, vorzugsweise von 0,9 bis 30 bar, insbesondere von 1 bis 10 bar vorgenommen.

In analoger Weise, wie für die Isomerisierung des Addukts II zum Enamin IV und dessen Hydrolyse oder Hydrierung zu n-Butyraldehyd und/oder n-Butanol in flüssiger Phase mit Heterogenkatalysatoren beschrieben, kann durch Zufuhr eines Gemisches, das bestimmte Mengen an Wasser und Wasserstoff enthält, als auch durch die Wahl des verwendeten Katalysators die Umsetzung in der Gasphase in der Weise gesteuert werden, daß der Reaktionsaustrag aus dieser Verfahensstufe n-Butyraldehyd und n-Butanol in dem gewünschten Produktverhältnis enthält.

Zur Aufarbeitung des gasförmigen Reaktionsaustrages kann dieser vorteilhaft, gegebenenfalls nach Entspannung auf Atmosphärendruck, direkt in eine Destillationsapparatur eingeleitet und dort destillativ in seine Bestandteile aufgetrennt werden.

Als Katalysatoren für die Isomerisierung des Addukts II zum Enamin IV und dessen Hydrolyse oder Hydrierung zu n-Butyraldehyd und/oder n-Butanol in der Gasphase in einer einzigen Verfahrensstufe können grundsätzlich die gleichen Heterogenkatalysatoren eingesetzt werden, wie sie auch bei der gleichen Umsetzung in flüssiger Phase Anwendung finden. Vorzugsweise werden im Gasphasenverfahren rein anorganische, mineralische Katalysatoren verwendet. Bevorzugte Katalysatoren sind beispielsweise Trägerkatalysatoren, die Elemente der I., VI., VII und/oder VIII. Nebengruppe, gegebenenfalls in Kombination mit einem oder mehreren Elementen der V. Nebengruppe, des Periodensystems der Elemente, insbesondere der Elemente der I., VII. und VIII. Nebengruppe des Periodensystems der Elemente auf einem Brönsted- oder Lewis-acide wirkenden Trägermaterial abgeschieden enthalten. Besonders vorteilhafte Katalysatoren sind z.B. Rhenium auf Titandioxid, Rhenium auf Siliziumdioxid, Kupfer auf Aktivkohle, Kupfer auf Siliziumdioxid, Kupfer auf Kieselgur, Kupfer auf Kieselgel, Kupfer auf Titandioxid, Kupfer auf Zirkondioxid, Kupfer auf Magnesiumsilikat, Kupfer auf Aluminiumsilikat, Kupfer auf Bleicherde, Kupfer auf Zeolith, Ruthenium auf Aktivkohle, Ruthenium auf Siliziumdioxid, Ruthenium auf Aluminiumoxid, Ruthenium auf Zirkoniumdioxid, Ruthenium auf Magnesiumoxid und Ruthenium auf Titandioxid und mit Selen oder Blei partiell vergiftete Palladium auf Aktivkohle-Katalysatoren.

Die Isomerisierung des Addukts II zum Enamin IV und dessen Hydrolyse oder Hydrierung zu n-Butyraldehyd und/oder n-Butanol in einer einzigen Verfahrensstufe unter Verwendung von Heterogenkatalysatoren kann sowohl bei Anwendung des Flüssigphasenverfahrens als auch bei Anwendung des Gasphasenverfahrens dahingehend vorteilhaft weiter ausgestaltet werden, daß man bei Verwendung eines einzigen Festbetts zur Durchführung dieser Reaktionen eine kombinierte Katalysatorschüttung, bestehend aus mindestens 2 Schichten unterschiedlicher Heterogenkatalysatoren, einsetzt, die sich in Aktivität und gegebenenfalls Selektivität für die beiden Teilreaktionen c) und d) unterscheiden, so daß z.B. in der ersten, zum Einlaß des Reaktionsgutes in den Reaktor hin gelegenen Schicht zunächst das Addukt II mit hoher Aktivität und Selektivität zum Enamin IV isomerisiert wird, welcher dann beim Passieren der nächsten, zum Ausgang des Reaktors hin gelegenen Schicht oder hin gelegenen Schichten aus Katalysatoren mit geringerer Isomerisierungs-, dafür höherer Hydrolyse- und/oder Hydrieraktivität mit hoher Aktivität und Selektivität zu n-Butyraldehyd und/oder n-Butanol umgewandelt wird.

Durch die Aneinanderreihung mehrerer Schichten unterschiedlich aktiver und/oder selektiver Katalysatoren kann die Wärmeentwicklung bei der Hydrolyse bzw. der kombinierten Hydrolyse/Hydrierung des Enamins IV gezielt beeinflußt und dadurch die Gesamtselektivität der Umsetzung erhöht werden. Dieser Effekt kann weiter verstärkt werden, indem z.B. die Reaktanten Wasser und/oder Wasserstoff nicht gemeinsam mit dem Addukt II in den Reaktor eingeleitet, sondern getrennt vom Addukt II in die Zone der Katalysatorschüttung eingespeist werden, in der die Hydrolyse bzw. der kombinierten Hydrolyse/Hydrierung stattfindet. Das Wasser und der Wasserstoff können gemeinsam in die betreffenden Zonen der Katalysatorschüttung eingespeist werden oder aber auch einzeln in verschiedene Zonen der Katalysatorschüttung geleitet werden. Statt in einer kombinierten Schüttung können die verschiedenen, bei dieser Verfahrensausgestaltung für die einzelnen Teilreaktionen verwendeten Katalysatoren auch einzeln, auf mehrere Festbette verteilt, angeordnet sein.

Obgleich die Durchführung der Teilreaktionen c) und d) des erfindungsgemäßen Verfahrens in einer einzigen Verfahrensstufe, z.B. nach den zuvor beschriebenen Verfahrensweisen eine bevorzugte Ausführungsvorm des erfindungsgemäßen Verfahrens darstellt, kann es sich unter Umständen als vorteilhaft erweisen, die einzelnen Teilreaktionen, nämlich die Isomerisierung des Addukts II zum Enamin IV, die Hydrolyse des Enamins IV zu n-Butyraldehyd oder die Hydrierung des Butyraldehyds zu n-Butanol in mehreren Verfahrensstufen durchzuführen. Beispielsweise ist es möglich, jede einzelne dieser Reaktionen in einer Verfahrensstufe vorzunehmen, indem man zunächst in einer Verfahrensstufe das Addukt II zum Enamin IV isomerisiert, anschließend das Enamin IV zu n-Butyraldehyd hydrolysiert und dann den n-Butyraldehyd zu n-Butanol hydriert, oder den entstandenen Butyraldehyd oder ein Teil des entstandenen Butyraldehyds abtrennt, in einer weiteren Stufe aldolisiert und anschließend zu 2-Ethylhexanol hydriert. Diese Verfahrensschritte verstehen sich für den Fachmann von selbst. Ebenso kann die Isomerisierung des Addukts II zum Enamin IV für sich in einer Verfahrensstufe durchgeführt werden und das Enamin IV anschließend zu n-Butyraldehyd hydrolysiert oder in einer Hydrolyse-/Hydrierumsetzung zu n-Butanol oder einer Mischung aus n-Butanol und n-Butyraldehyd verarbeitet werden. Eine weitere Variante des erfindungsgemäßen Verfahrens besteht darin, die Isomerisierung des Addukts II zum Enamin IV und dessen Hydrolyse zu n-Butyraldehyd in einer einzigen Verfahrensstufe auszuführen und anschließend den hierbei erhaltenen n-Butyraldehyd in einer weiteren Verfahrensstufe zu n-Butanol zu hydrieren.

Bei der Aufteilung der Teilreaktionen c) und d) auf mehrere Verfahrensstufen können in den einzelnen Verfahrensstufen verschiedenerlei Betriebsweisen angewandt werden. Beispielsweise kann die Isomerisierung des Addukt II zum Enamin IV wahlweise unter homogener Katalyse oder an Heterogenkatalysatoren vorgenommen werden, desgleichen kann die Hydrolyse bzw. die kombinierte Hydrolyse/Hydrierung des Enamin IV zu n-Butyraldehyd und/oder n-Butanol wahlweise in flüssiger Phase unter Verwendung von Homogenkatalysatoren oder Heterogenkatalysatoren oder in der Gasphase ausgeführt werden.

Bei der Aufteilung der einzelnen Teilreaktionen c) und d) auf mehrere Verfahrensstufen ist es auch möglich, anstelle der zuvor beschriebenen Katalysatoren, welche sowohl die Isomerisierung des Addukts II zum Enamin IV als auch dessen Hydrolyse und Hydrierung katalysieren, in den einzelnen Verfahrensstufen Katalysatoren zu verwenden, welche nur die jeweilige Teilreaktion katalysieren können. So kann das Enamin IV beispielsweise mittels Brönsted-Säure-Katalysatoren, wie Mineralsäuren, z.B. Halogenwasserstoffsäuren, Schwefelsäure, verdünnter Salpetersäure, Phosphorsäure oder heterogenen Brönsted-Säuren, wie Ionenaustauschern, Zeolithen, Bleicherden oder sauren Phosphaten, beispielsweise Aluminiumphosphate, zu n-Butyraldehyd hydrolysiert werden. Für diesen Fall wird das Amin aus seinem Säuresalz durch Zugabe von Basen wieder freigesetzt.

Das bei der Hydrolyse oder kombinierten Hydrolyse/Hydrierung aus dem Enamin IV freigesetzte Amin R¹R²NH I wird vorzugsweise wieder in die Umsetzung gemäß Teilreaktion a) zurückgeführt. Aufgrund der Möglichkeiten, die Teilreaktionen der Isomerisierung des Addukts II zum Enamin IV und dessen Hydrolyse bzw. dessen kombinierte Hydrolyse/Hydrierung in mehrere Verfahrensschritte aufzuteilen, erhält man eine höhere Flexibilität beim Bau einer Anlage zur Durchführung des erfindungsgemäßen Verfahrens, wodurch erhebliche Einsparungen möglich sind.

Der erfindungsgemäß hergestellte n-Butyraldehyd kann nach beispielsweise destillativer Isolierung in an sich bekannter Weise zu 2-Ethylhexanol umgesetzt werden. So kann n-Butyraldehyd bei 80 bis 130°C und 3 bis 10 bar Druck in Gegenwart von Natrium- oder Kaliumhydroxid in das Aldolprodukt 2-Ethylhex-2-enal überführt werden, das dann katalytisch bei etwa 200 bis 250°C und 50 bis 200 bar Wasserstoffdruck zu 2-Ethylhexanol reduziert werden kann.

Es ist auch möglich, ein erfindungsgemäß erhaltenes, n-Butyraldehyd enthaltendes Reaktionsgemisch in der oben angegebenen Weise einer Aldolreaktion und einer Hydrierung zu unterwerfen und anschließend das Produkt von den im ursprünglichen Reaktionsgemisch enthaltenen Verunreinigungen destillativ zu reinigen.

Bevorzugt wird eine Verfahrensvariante, in der der Butyraldehyd durch saure Hydrolyse des Enamins IV hergestellt wird, wie es oben ausführlich beschrieben wurde. In Gegenwart der im Reaktionsgemisch befindlichen Säure kann der n-Butyraldehyd zum Aldolprodukt 2-Ethylhex-2-enal reagieren. Während man diese Reaktion zu unterdrücken versucht, wenn n-Butyraldehyd als Produkt gesucht ist, kann man die Reaktion so steuern, daß die Aldolreaktion bevorzugt eintritt. Dazu sind in der Regel unter sonst gleichen Reaktionsbedingungen längere Reaktionszeiten notwendig. Es ist auch möglich, durch Erhöhung der Temperatur oder der Säurekonzentration im Vergleich zu einer auf hohe Ausbeute an n-Butyraldehyd abzielenden Verfahrensweise eine schnellere Reaktion zum Aldolprodukt zu erzielen. Aufgrund der vielfältigen Variationsmöglichkeiten bei der Herstellung des n-Butyraldehyds muß der Fachmann durch Vorversuche die zur Erzielung einer hohen Ausbeute an Aldolprodukt idealen Reaktionsbedingungen ermitteln. Das so erhaltene Aldolprodukt kann in bekannter Weise zum 2-Ethylhexanol hydriert werden. Ganz besonders vorteilhaft verläuft die Gesamtreaktion zu 2-Ethylhexanol, wenn in Verfahrensschritt c) des erfindungsgemäßen Verfahrens zur Herstellung von n-Butyraldehyd und/oder n-Butanol ein homogener Katalysator, z.B. ein Rutheniumkatalysator, eingesetzt wird, der gleichzeitig die in Verfahrensschritt d) vorgesehene Hydrierung erlaubt, wenn weiterhin die Hydrolyse des Enamins IV in Gegenwart einer Säure durchgeführt wird und ansonsten in Verfahrensschritt d) in Gegenwart von Wasserstoff gearbeitet wird, wie es für die Herstellung von n-Butanol erläutert wurde. In diesem Fall kann die Reaktion von Enamin IV zu 2-Ethylhexanol in einer Stufe vorgenommen werden. Die Aufarbeitung und Rückführung des Amins I geschieht in analoger Weise, wie es bei der Herstellung von n-Butanol ausgeführt wurde.

### Verfahrensschritt a)

### Beispiele 1 - 7

Ein 0,3 l Stahlautoklav wurde mit 0,50 mol des entsprechenden Amins, 1,25 mol Palladiumacetylacetonat und 2,5 mmol Phosphinligand befüllt und anschließend die gewählte Menge Butadien aufgepreßt. Das Reaktionsgemisch wurde bei 145°C unter Eigendruck gerührt. Nach Beendigung der Reaktion wurde der Flüssigkeitsaustrag gaschromatographisch (Carbowax 20 M, 2 m, (Flächenprozent bezogen auf Amin)) untersucht.

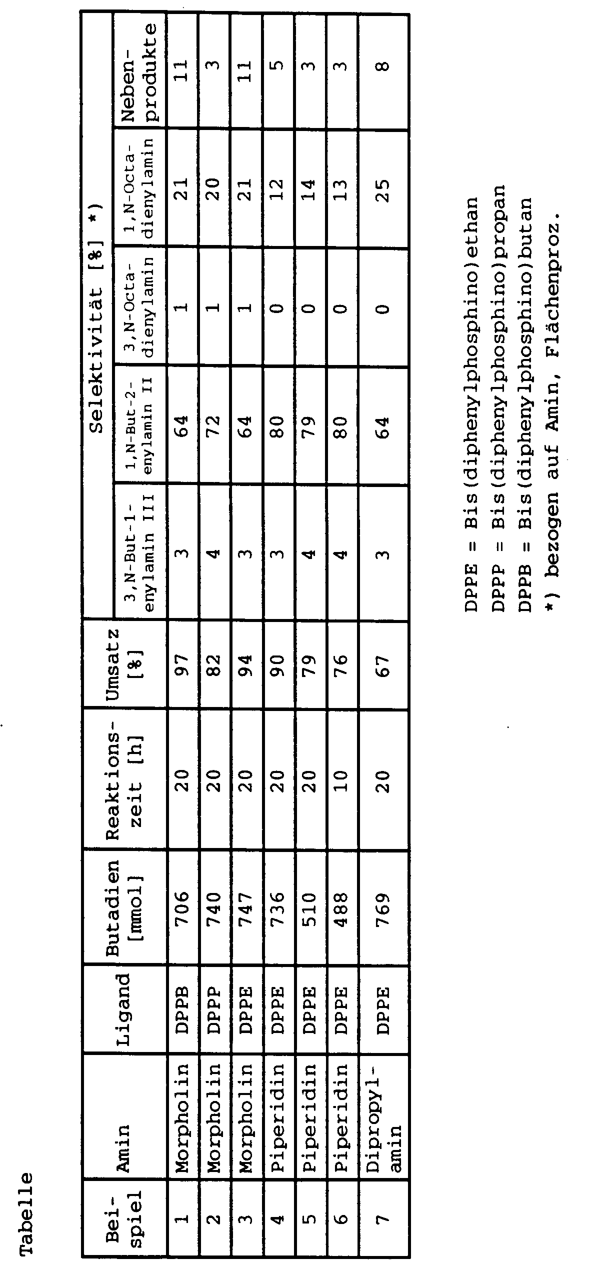

### Beispiel 8

Zu einer Lösung aus 0,12 g (0,25 mmol) [Bis(di-tert.-butylphosphino)methan]palladiumdichlorid in 4,36 g (50 mmol) Morpholin wurden nacheinander 0,107 g (0,55 mmol) AgBF₄ und 0,076 g (0,25 mmol) Bis(di-tert.-butylphosphino)methan hinzugegeben und anschließend 2,70 g (50 mmol) Butadien aufgepreßt. Nach 5 h Reaktionszeit bei 80°C und Eigendruck wurde nach gaschromatographischer Analyse ein Umsatz von 75 % mit einer Selektivität von 1 Fl.% Octatrien, 6 Fl.% (3,N)-(But-1-enyl)-morpholin, 76 Fl.% (1,N)-(But-2-enyl)morpholin, 10 Fl.% Octadienylmorpholin und 1 Fl.% Nebenprodukte erhalten.

### Beispiel 9

Eine Lösung von 8,70 g (100 mmol) Morpholin, 0,076 (0,25 mmol) Pd(acac)₂ (acac = Acetylacetonat), 0,35 g (0,875 mmol) DPPE wurde mit 0,141 g p-Toluolsulfonsäure und 2,4 g Methanol versetzt. Nach Aufpressen von 5,4 g (100 mmol) Butadien wurde 17 h bei 100°C unter Eigendruck gerührt. Man erhält nach gaschromatografischer Analyse einen Umsatz von 96 % mit einer Selektivität von 1 Fl.% Octatrien, 3 Fl.% (3,N)-(But-1-enyl)morpholin, 94 Fl.% (1,N)-(But-2-enyl)-morpholin, 1 Fl.% Octadienylamin und 1 Fl.% an Nebenrodukten.

### Verfahrensschritt c)

### Beispiel 10

Eine Lösung von 3,06 g (21,7 mmol) (1,N)-(But-2-enyl)-morpholin, 0,023 g (0,024 mmol) HRuCl(CO)(PPh₃)₃ und 0,034 g (0,125 mmol) Triphenylphosphin wurde mit 20 g Wasser versetzt und bei 12 bar Wasserstoffdruck und 150°C gerührt. Nach 20 h Reaktionszeit wurde nach gaschromatographischer Analyse ein Umsatz von 90 % mit einer Selektivität von 64 Fl.% 1-Butanol, 4 Fl.% (1,N)-(But-1-enyl)morpholin und 32 % N-Butylmorpholin erhalten. Das Morpholin wurde zurückgewonnen.

### Beispiel 11

Unter Argon wurden 19 g (122 mmol) (1,N)-(But-2-enyl)-morpholin in einer Schmelze von 30 g (114 mmol) Triphenylphosphin und 1,5 g (1,63 mmol) HRh(PPh₃)₃CO 245 min bei 120°C unter Rühren umgesetzt. Anschließend wurden die Produkte bei vermindertem Druck (15 mbar) bis 120°C abdestilliert und gaschromatographisch analysiert. Es wurde folgendes Ergebnis erzielt: Der Umsatz an (1,N)-(But-2-enyl)-morpholin 98,1 %, neben (1,N)-(But-1-enyl)morpholin war kein weiteres Produkt nachweisbar (Selektivität 100 %).

### Verfahrensschritt d)

### Beispiel 12

2 g (15,9 mmol) (1,N)-(But-1-enyl)-morpholin wurde mit 5 g (278 mmol) Wasser in Gegenwart von 14 g 1,4-Dioxan und 0,5 g Bayer-Kat. 2611 (saurer Ionentauscher) 4 h auf 120°C erhitzt. Das zunächst aus zwei Flüssigphasen bestehende Gemisch wird einphasig. Die GC-Analyse zeigte einen Umsatz von 75 % mit einer Selektivität von 31 Fl.% n-Butyraldehyd und 69 Fl.% 2-Ethyl-hex-2-enal. Morpholin wurde zurückerhalten.

### Verfahrensschritte c) und d)

### Beispiel 13

In einem Reaktor wurden 30 g (50 ml) eines Kupfer auf Kieselgel tragenden Katalysators, der einen Kupfergehalt, berechnet als CuO, von 26 Gew.-% hatte, eingefüllt und der Katalysator innerhalb 18 h mit Formiergas (5 % Wasserstoff, 95 % Stickstoff) bei Atmosphärendruck und einer Temperatur von anfangs 30°C und bis zu einer Endtemperatur von 190°C aktiviert. Nach der Aktivierung erfolgte die Umstellung des Gases auf reinen Wasserstoff.

Danach wurde über den auf 190°C temperierten Reaktor und über einen auf 150°C erhitzten Vorheizer 1,5 g/h Wasser und 2,75 g/h (1,N)-(But-2-enyl)-morpholin bei Atmosphärendruck geleitet. Gleichzeitig wurde dem Reaktor ein Wasserstoffstrom von 8 l/h zugeführt. Nach Abkühlung wurde der einphasige, flüssige Reaktoraustrag gaschromatrographisch analyisert. Bei einem Umsatz von 60 % wurde eine Selektivität von 54 Fl.% 1-Butanol, 4 Fl.% (1,N)-(But-1-enyl)-morpholin und 42 Fl.% N-Butylmorpholin erzielt.

## Patentansprüche

1. Verfahren zur Herstellung von n-Butyraldehyd und/oder n-Butanol, dadurch gekennzeichnet, daß man
a) 1,3-Butadien mit einem Amin der Formel I
R¹R²NH I,
in der die Reste unabhängig voneinander für Wasserstoff, gegebenenfalls substituierte aliphatische oder cycloaliphatische Reste, Aryl- oder Aralkylreste stehen oder zu einem Brückenglied, das Heteroatome tragen kann, verbunden sind, bei erhöhter Temperatur und bei erhöhtem Druck in Gegenwart einer Verbindung eines Elementes aus der Gruppe VIIIb des Periodensystems der Elemente, in Gegenwart eines Alkalimetallamids oder in Gegenwart eines basischen Metalloxids zu einem Gemisch der Addukte der Formeln II und III 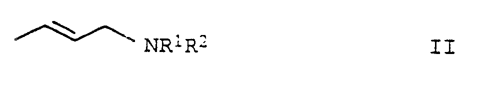 umsetzt, 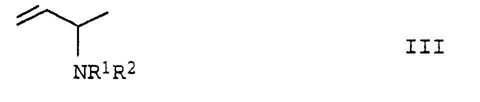
b) das Addukt III, zum Addukt II isomerisiert,
c) das Addukt II in Gegenwart eines homogenen oder heterogenen Übergangsmetallelement-Katalysators in flüssiger Phase oder in Gegenwart eines heterogenen, Übergangsmetallelement-haltigen Katalysators in der Gasphase zum Enamin der Formel IV 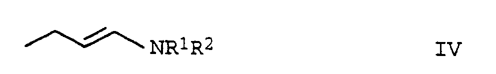 isomerisiert, und d) aus diesem Enamin IV durch dessen Umsetzung mit Wasserstoff und Wasser oder Wasser in Gegenwart eines homogenen oder heterogenen Übergangsmetallelement-Katalysators in flüssiger Phase, in Gegenwart eines heterogenen Übergangsmetallelement-haltigen Katalysators in der Gasphase, in Gegenwart einer Säure oder in Gegenwart eines der genannten Katalysatoren und einer Säure n-Butyraldehyd und/oder n-Butanol erzeugt, das Amin I freisetzt und das freigesetzte Amin I wieder in die Umsetzung gemäß Teilreaktion a) zurückführt.

2. Verfahren nach Anspruch 1, dadurch gekennzeichnet, daß man die Umsetzung von 1,3-Butadien mit einem Amin R¹R²NH I in Gegenwart eines Katalysators aus einem Alkyl-, Aryl- oder Arylalkyl-Phosphin-Komplex des Rhodiums, Rutheniums, Nickels, Palladiums, Iridiums oder Platins durchführt.

3. Verfahren nach Anspruch 1 oder 2, dadurch gekennzeichnet, daß man als Amin I Morpholin, Piperidin oder Dipropylamin verwendet.

4. Verfahren nach den Ansprüchen 1 bis 3, dadurch gekennzeichnet, daß man das Addukt III vom Addukt II abtrennt und das Addukt III anschließend in die Teilreaktion a) zurückführt und dort zum Addukt II isomerisiert.

5. Verfahren nach den Ansprüchen 1 bis 4, dadurch gekennzeichnet, daß man die Teilreaktionen c) - Isomerisierung des Addukts II zum Enamin IV - und d) - Hydrolyse oder kombinierte Hydrolyse/Hydrierung des Enamins IV zu n-Butyraldehyd und/oder n-Butanol - in einer einzigen Verfahrensstufe durchführt.

6. Verfahren nach den Ansprüchen 1 bis 5, dadurch gekennzeichnet, daß man die Teilreaktion c) und d) in Gegenwart eines heterogenen Katalysators, der Kupfer enthält, durchführt.

7. Verfahren nach den Ansprüchen 1 bis 5, dadurch gekennzeichnet, daß man die Teilreaktionen c) und d) in flüssiger Phase in Gegenwart eines homogen im Reaktionsmedium löslichen Katalysators aus einem ein- oder mehrzähnigen Phosphin- oder Phosphit-Komplex eines Elementes der Gruppen Ib, VIb, VIIb und VIIIb des Periodensystems der Elemente vornimmt.

8. Verfahren nach den Ansprüchen 1 bis 4, dadurch gekennzeichnet, daß man die Teilreaktion d) in Gegenwart von sauren Ionentauschern vornimmt.

9. Verfahren zur Herstellung von 2-Ethylhexanol, dadurch gekennzeichnet, daß man
a) 1,3-Butadien mit einem Amin der Formel I
R¹R²NH I,
in der die Reste unabhängig voneinander für Wasserstoff, gegebenenfalls substituierte aliphatische oder cycloaliphatische Reste, Aryl- oder Aralkylreste stehen oder zu einem Brückenglied, das Heteroatome tragen kann, verbunden sind, bei erhöhter Temperatur und bei erhöhtem Druck in Gegenwart einer Verbindung eines Elementes aus der Gruppe VIIIb des Periodensystems der Elemente, in Gegenwart eines Alkalimetallamids oder in Gegenwart eines basischen Metalloxids, zu einem Gemisch der Addukte der Formeln II 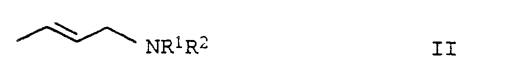 und III 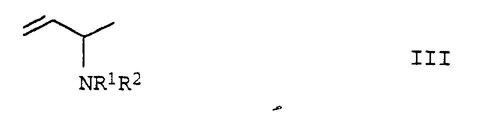 umsetzt,
b) gegebenenfalls das Addukt III zum Addukt II isomerisiert,
c) das Addukt II in Gegenwart eines homogenen Übergangsmetallelement-Katalysators in flüssiger Phase zum Enamin IV 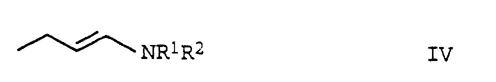 isomerisiert,
e) aus diesem Enamin IV durch Umsetzung mit Wasserstoff und Wasser in Gegenwart des in Teilreaktion c) eingesetzten Katalysators sowie in Gegenwart einer Säure n-Butyraldehyd erzeugt,
f) den n-Butyraldehyd zum Aldolprodukt 2-Ethylhex-2-enal umsetzt,
g) das Aldolprodukt zu 2-Ethylhexanol hydriert, das Amin I wieder freisetzt und das freigesetzte Amin I wieder in die Umsetzung gemäß Teilreaktion a) zurückführt.

## Claims

1. A process for the preparation of n-butyraldehyde and/or n-butanol, wherein
a) 1,3-butadiene is reacted with an amine of the formula I
R¹R²NH I
where the radicals, independently of one another, are each hydrogen, an unsubstituted or substituted aliphatic or cycloaliphatic radical, aryl or aralkyl or are linked to form a bridge member which may carry heteroatoms, at elevated temperature and at superatmospheric pressure in the presence of a compound of an element of group VIIIb of the Periodic Table of Elements, in the presence of an alkali metal amide or in the presence of a basic metal oxide to give a mixture of the adducts of the formulae II 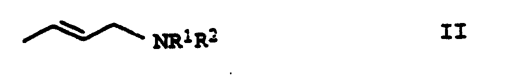 and III 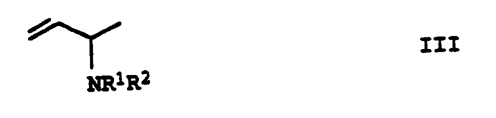
b) the adduct III is isomerized to the adduct II,
c) the adduct II is isomerized in the presence of a homogeneous or heterogeneous transition metal catalyst in the liquid phase or in the presence of a heterogeneous transition metal-containing catalyst in the gas phase to give an enamine of the formula IV 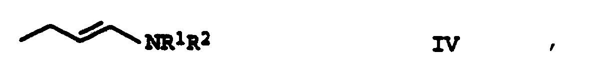 and
d) n-butyraldehyde and/or n-butanol are produced from this enamine IV by reacting it with hydrogen and water or water in the presence of a homogeneous or heterogeneous transition metal catalyst in the liquid phase, in the presence of a heterogeneous transition metal-containing catalyst in the gas phase, in the presence of an acid or in the presence of one of the stated catalysts and of an acid, the amine I is liberated and the liberated amine I is recycled to the reaction according to reaction step a).

2. A process as claimed in claim 1, wherein the reaction of 1,3-butadiene with an amine R¹R²NH I is carried out in the presence of a catalyst comprising an alkyl-, aryl- or arylalkyl-phosphine complex of rhodium, of ruthenium, of nickel, of palladium, of iridium or of platinum.

3. A process as claimed in claim 1 or 2, wherein the amine I used is morpholine, piperidine or dipropylamine.

4. A process as claimed in any of claims 1 to 3, wherein the adduct III is separated from the adduct II, and the adduct III is then recycled to the reaction step a) and isomerized there to give the adduct II.

5. A process as claimed in any of claims 1 to 4, wherein the reaction steps c) - isomerization of the adduct II to the enamine IV - and d) - hydrolysis or combined hydrolysis/hydrogenation of the enamine IV to n-butyraldehyde and/or n-butanol - are carried out in a single process stage.

6. A process as claimed in any of claims 1 to 5, wherein the reaction steps c) and d) are carried out in the presence of a heterogeneous catalyst which contains copper.

7. A process as claimed in any of claims 1 to 5, wherein the reaction steps c) and d) are carried out in the liquid phase in the presence of a catalyst which dissolves homogeneously in the reaction medium and comprises a monodentate or polydentate phosphine or phosphite complex of an element of groups Ib, VIb, VIIb and VIIIb of the Periodic Table of Elements.

8. A process as claimed in any of claims 1 to 4, wherein the reaction step d) is carried out in the presence of acidic ion exchangers.

9. A process for the preparation of 2-ethylhexanol, wherein
a) 1,3-butadiene is reacted with an amine of the formula I
R¹R²NH I
where the radicals, independently of one another, are each hydrogen, an unsubstituted or substituted aliphatic or cycloaliphatic radical, aryl or aralkyl or are linked to form a bridge member which may carry heteroatoms, at elevated temperatures and at superatmospheric pressure in the presence of a compound of an element of group VIIIb of the Periodic Table of Elements, in the presence of an alkali metal amide or in the presence of a basic metal oxide to give a mixture of the adducts of the formulae II 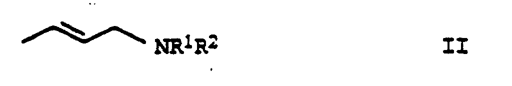
and III 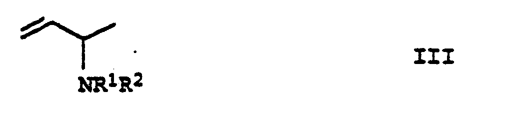
b) if required, the adduct III is isomerized to the adduct II,
c) the adduct II is isomerized in the presence of a homogeneous transition metal catalyst in the liquid phase to give the enamine IV 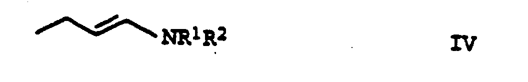
e) n-butyraldehyde is produced from this enamine IV by reaction of hydrogen and water in the presence of the catalyst used in reaction step c) and in the presence of an acid,
f) the n-butyraldehyde is converted into the aldol product 2-ethylhex-2-enal, and
g) the aldol product is hydrogenated to 2-ethylhexanol, the amine I is liberated again and the liberated amine I is recycled to the reaction according to reaction step a).

## Revendications

1. Procédé de préparation de n-butyraldéhyde et/ou de n-butanol, caractérisé en ce que
a) on fait réagir du 1,3-butadiène avec une amine de formule I
R¹R²NH I,
dans laquelle les radicaux, indépendamment l'un de l'autre, représentent chacun un atome d'hydrogène, un radical aliphatique ou cycloaliphatique éventuellement substitué, un radical aryle ou aralkyle, ou encore sont reliés à un chaînon pontant pouvant porter des hétéroatomes, à haute température et sous pression élevée, en présence d'un composé d'un élément du groupe VIII de la classification périodique des éléments, en présence d'un amidure d'un métal alcalin ou en présence d'un oxyde métallique basique, pour donner un mélange des produits d'addition ayant les formules II 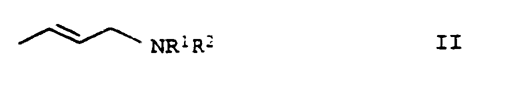 et III 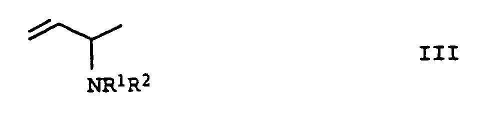
b) on isomérise le produit d'addition III en le produit d'addition II,
c) on isomérise le produit d'addition II, en phase liquide en présence d'un catalyseur homogène ou hétérogène à base d'un élément de la série des métaux de transition, ou en phase gazeuse en présence d'un catalyseur hétérogène contenant un élément de la série des métaux de transition, pour obtenir l'énamine de formule IV et 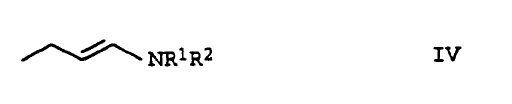
d) à partir de cette énamine IV, par réaction de cette dernière avec de l'hydrogène et de l'eau, ou avec de l'eau, en phase liquide en présence d'un catalyseur homogène ou hétérogène a base d'un élément de la série des métaux de transition, en phase gazeuse en présence d'un catalyseur hétérogène contenant un élément de la série des métaux de transition, en présence d'un acide, ou en présence de l'un des catalyseurs mentionnés et d'un acide, on produit du n-butyraldéhyde et/ou du n-butanol, on libère l'amine I et on renvoie l'amine libérée I dans la réaction selon la réaction partielle a).

2. Procédé selon la revendication 1, caractérisé en ce qu'on met en oeuvre la réaction du 1,3-butadiène avec une amine R¹R²NH I en présence d'un catalyseur à base d'un complexe alkyl-, aryl- ou arylalkyl-phosphinique du rhodium, du ruthénium, du nickel, du palladium, de l'iridium ou du platine.

3. Procédé selon la revendication 1 ou 2, caractérisé en ce qu'en tant qu'amine I on utilise la morpholine, la pipéridine ou la dipropylamine.

4. Procédé selon les revendications 1 à 3, caractérisé en ce qu'on sépare le produit d'addition III du produit d'addition II, puis on renvoie le produit d'addition III dans la réaction partielle a) et on l'y isomérise en le produit d'addition II.

5. Procédé selon les revendications 1 à 4, caractérisé en ce qu'on met en oeuvre les réactions partielles c) (isomérisation du produit d'addition II en l'énamine IV) et d) (hydrolyse ou combinaison hydrolyse/hydrogénation de l'énamine IV en n-butyraldéhyde et/ou n-butanol) en une étape unique.

6. Procédé selon les revendications 1 à 5, caractérisé en ce qu'on met en oeuvre la réaction partielle c) et d) en présence d'un catalyseur hétérogène contenant du cuivre.

7. Procédé selon l'une des revendications 1 à 5, caractérisé en ce qu'on met en oeuvre les réactions partielles c) et d) en phase liquide en présence d'un catalyseur homogène soluble dans le milieu réactionnel, à base d'un complexe mono- ou polydenté phosphinique ou phosphitique d'un élément des groupes Ib, VIb, VIIb et VIII de la classification périodique des éléments.

8. Procédé selon les revendications 1 à 4, caractérisé en ce qu'on met en oeuvre la réaction partielle d) en présence d'échangeurs d'ions acides.

9. Procédé de préparation de 2-éthylhexanol, caractérisé en ce que
a) on fait réagir du 1,3-butadiène avec une amine de formule I
R¹R²NH I,
dans laquelle les radicaux, indépendamment l'un de l'autre, représentent chacun un atome d'hydrogène, un radical aliphatique ou cycloaliphatique éventuellement substitué, un radical aryle ou aralkyle, ou encore sont reliés à un chaînon pontant pouvant porter des hétéroatomes, à haute température et sous pression élevée, en présence d'un composé d'un élément du groupe VIII de la classification périodique des éléments, en présence d'un amidure d'un métal alcalin ou en présence d'un oxyde métallique basique, pour donner un mélange des produits d'addition ayant les formules II et III 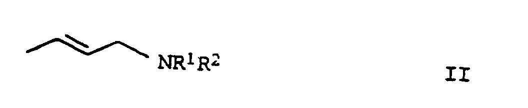 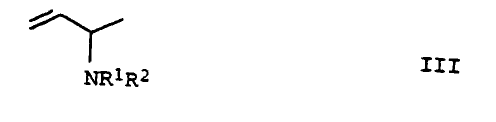
b) éventuellement, on isomérise le produit d'addition III en le produit d'addition II,
c) on isomérise le produit d'addition II en présence d'un catalyseur homogène en phase liquide à base d'un élément de la série des métaux de transition, pour obtenir l'énamine IV 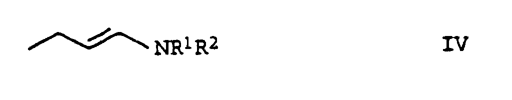
e) à partir de cette énamine IV, par réaction avec de l'hydrogène et de l'eau, en présence du catalyseur utilisé dans la réaction partielle c) et en présence d'un acide, on produit du n-butyraldéhyde,
f) on convertit le n-butyraldéhyde en le produit aldol 2-éthylhex-2-énal,
g) on hydrogène le produit aldol en 2-éthylhexanol, on libère de nouveau l'amine I et on renvoie l'amine libérée I dans la réaction selon la réaction partielle a).
